# FASCICULE DE BREVET EUROPEEN

(11) **EP 1 435 993 B1**
(45) Date de publication et mention de la délivrance du brevet: **27.07.2011**
(21) Numéro de dépôt: 02774915.9
(22) Date de dépôt: 09.09.2002
(51) Int. Cl.: A61K 31/7088, A61K 39/395, A61K 38/17, G01N 33/68, C07K 14/47, A61P 25/00, A61P 37/00

(54) **UTILISATION D'UNE PROTEINE DE LA FAMILLE DES CRMPS POUR LE TRAITEMENT DES MALADIES LIEES AU SYSTEME IMMUNITAIRE**
VERWENDUNG EINES PROTEINS DER CRMP FAMILIE ZUR BEHANDLUNG VON KRANKHEITEN DES IMMUNSYSTEMS
UTILISATION D'UNE PROTEINE DE LA FAMILLE DES CRMPS POUR LE TRAITEMENT DES MALADIES LIEES AU SYSTEME IMMUNITAIRE

(30) Priorité: 07.09.2001 FR 0111627; 16.10.2001 FR 0113342
(43) Date de publication de la demande: 14.07.2004
(73) Titulaire: INSTITUT NATIONAL DE LA SANTE ET DE LA RECHERCHE MEDICALE (INSERM), 75013 Paris (FR)
(72) Inventeur: GIRAUDON, Pascale, F-69005 Lyon (FR); BELIN, Marie-Françoise, F-69002 Lyon (FR); MALCUS, Christophe, F-69530 Brignais (FR); COLAS, Pierre, F-69370 Saint Didier au Mont d'Or (FR); ANTOINE, Jean-Christophe, F-42600 Chalain le Comtal (FR); HONNORAT, Jérôme, F-69500 Bron (FR)
(74) Mandataire: Neyret, Daniel Jean Marie
(86) Numéro de dépôt international: PCT/FR2002/003056
(87) Numéro de publication internationale: WO 2003/022298

(56) Documents cités:
- WO-A-98/30909
- WO-A-98/37192
- FR-A- 2 805 540
- US-A1- 2002 119 944
- YU ZHIYA ET AL: "CRMP-5 neuronal autoantibody: Marker of lung cancer and thymoma-related autoimmunity." ANNALS OF NEUROLOGY, vol. 49, no. 2, février 2001 (2001-02), pages 146-154, XP001075080 ISSN: 0364-5134 cité dans la demande
- GOSHIMA Y ET AL: "FUNCTIONS OF SEMAPHORINS IN AXON GUIDANCE AND NEURONAL REGENERATION" JAPANESE JOURNAL OF PHARMACOLOGY, vol. 82, no. 4, avril 2000 (2000-04), pages 273-279, XP001052708 ISSN: 0021-5198
- YOSHIDA HIROTAKA ET AL: "Collapsin response mediator protein-2 is associated with neurofibrillary tangles in Alzheimer's disease." JOURNAL OF BIOLOGICAL CHEMISTRY, vol. 273, no. 16, 17 avril 1998 (1998-04-17), pages 9761-9768, XP002199049 ISSN: 0021-9258 cité dans la demande
- LAPTEVA NATALIA ET AL: "Gene expression analysis in human monocytes, monocyte-derived dendritic cells, and alpha-galactosylceramide-pulsed monocyte-derived dendritic cells." BIOCHEMICAL AND BIOPHYSICAL RESEARCH COMMUNICATIONS, vol. 289, no. 2, 30 novembre 2001 (2001-11-30), pages 531-538, XP002199050 ISSN: 0006-291X

## Description

La présente demande concerne une nouvelle utilisation de protéines dites "CRMPs" (Collapsin Response Mediator Proteins) pour le diagnostic et la thérapie de pathologies liées à un dysfonctionnement du système immunitaire.

Les CRMPs, aussi connues sous les dénominations TOAD-64 (Minturn et al., 1995, J Neurosci 15:6757-6766), DRP (Dihydropyrimidinase related protein, Hamajima et al., 1996, Gene 180:157-163), C-22 (Quach et al., 1997, Mol Brain Res 46:329-332) ou ULIP (Unc-33-Like protein, Byk et al., 1998, Eur J Blochem 254:14-24 et demande internationale WO 98/37 192), sont des molécules de signalisation intracellulaire jusqu'ici reconnues comme spécifiques du système nerveux. Ces protéines ont été notamment décrites comme étant impliquées dans le contrôle du développement neuronal et la croissance axonale.

A ce jour, cinq protéines de cette famille sont identifiées, CRMP1, CRMP2, CRMP3, CRMP4 et CRMP5.

Certains membres de la famille ont été associés avec des désordres neurodégénératifs humains. Dans la maladie d'Alzheimer, on observe ainsi des niveaux élevés de CRMP2 phosphorylés en association avec des plaques de neurofibrilles (Yoshida et al., 1998, J Biol Chem 273:9761-9768).

Dans les syndromes neurologiques paranéoplasiques (SNP), qui sont des désordres neurodégénératifs, des patients développent des auto-anticorps (anticorps anti-CV2) reconnaissant les protéines CRMP (Honnorat et al., 1999, Eur J Neurosci 11:4226-4232).

Par suite, il a été proposé d'utiliser ces protéines dans le diagnostic et la thérapie des cancers et des SNP (FR 2 805 540 et WO 98137192).

Yu et al., 2001 (Annals of Neurology, 49(2) :146-154) rapporte par ailleurs la présence d'auto-anticorps neuronaux anti-CRMP5 dans des cas de cancer du poumon et de thymome.

De manière inattendue, les inventeurs ont mis en évidence la présence des CRMP dans les lymphocytes T. Plus particulièrement, ils ont montré des CRMPs 1, 2 et 4 à des taux élevés dans les lymphocytes T chez des patients atteints de pathologies dysimmunes. En l'occurrence, il apparaît que la présence de ces protéines y est accrue lorsqu'elle est associée à une anomalie de mort ou de prolifération des lymphocytes.

Par ailleurs, les inventeurs ont également observé une translocation nucléaire très augmentée de la CRMP2 des lymphocytes infectés par HTLV-1 et les rendant probablement hyperprolifératifs, ou de lymphocytes T de patients présentant une déficience immune liée au système Fas-Fas ligand. Cette translocation correspondrait à une forme très phosphorylée de CRMP2, plus particulièrement reconnue par son anticorps spécifique.

Enfin, comme il ressort des exemples ci-après, la présence de ces protéines a été également caractérisée dans des cellules épithéliales thymiques dès le stade embryonnaire. Cette découverte de CRMPs dans les cellules épithéliales immatures est un des rares exemples de protéine de signalisation intracellulaire dans le thymus.

Par ailleurs, la disparition des CRMPs dans le thymus adulte et leur induction dans les cellules T après stimulation du TCR indique une relation avec le "réarrangement" du récepteur des cellules T, TCR, et l'éducation des thymocytes.

En conséquence, l'ensemble de ces nouvelles données témoigne de l'intervention des protéines CRMPs dans une voie de signalisation qui conduit à la prolifération, la mort, la maturation et l'éducation des lymphocytes et donc de leur implication dans la régulation de la réponse immunitaire.

Ces observations sont d'autant plus inattendues que, jusqu'à ce jour, les CRMPS n'étaient considérées surtout sur un plan thérapeutique que comme des cibles potentielles pour traiter des pathologies du système nerveux central (SNC).

Il est clair que cette découverte ouvre de nouvelles perspectives thérapeutiques. Il est désormais envisageable d'agir sur la prolifération ou la mort de cellules T et/ou d'agir sur les cellules du thymus et l'auto-immunité via une intervention au niveau des protéines CRMPs dans les lymphocytes.

En l'occurrence, la présente demande propose d'utiliser les CRMPs comme cibles pour le traitement, le pronostic et/ou le diagnostic de pathologies liées à un dysfonctionnement du système immunitaire et notamment de la prolifération des cellules T.

Il s'agit en particulier de moduler l'expression ou l'activité d'une ou plusieurs CRMPs, de façon à moduler l'apoptose, la prolifération ou la migration des cellules.

Ainsi, une altération des CRMPs, comme par exemple une modification de la phosphorylation de CRMP2, est susceptible de conduire à une hyperprolifération ou à une mort cellulaire.

De même, une stimulation des CRMPs semble constituer un moyen efficace pour restaurer une signalisation déficiente et améliorer la survie lymphocytaire comme dans le cas de l'infection par le VIH.

Enfin, un contrôle de l'hyperexpression des CRMPs semble déterminant pour réduire la prolifération de lymphocytes rendus "hyper" prolifératifs comme dans le cas d'une infection par HTLV-1 ou une mutation de Fas. Cette inhibition pourrait aussi améliorer les processus autoimmuns qui impliquent des lymphocytes T reconnaissant des déterminants du soi (autoréactifs), et les atteintes du SNC liées à l'invasion du SNC par ces lymphocytes T (TSP/HAM liée à HTLV-1, encéphalite liée à la rougeole, sclérose en plaques).

Selon certains cas, on cherche donc plutôt à augmenter ou stimuler l'expression ou l'activité des CRMPs (par exemple dans le cas d'une infection des lymphocytes par le virus VIH du SIDA). Dans d'autres cas en revanche, on cherche à réprimer ou inhiber l'expression ou l'activité des CRMP, par exemple pour réduire la prolifération ou la migration des lymphocytes par exemple dans le cas d'un lymphome.

Bloquer l'activité CRMP peut également être avantageuse pour bloquer l'effet de la protéine prion PrP pathologique.

La demande décrit tout d'abord l'utilisation d'au moins une protéine de la famille des CRMPs, fragment polypeptidique ou dérivé biologiquement actif de celle-ci, ou d'une séquence ou fragment de séquence nucléotidique codant pour ladite protéine pour la fabrication d'un médicament destiné à traiter des pathologies liées à un dysfonctionnement du système immunitaire.

La demande décrit ensuite l'utilisation d'une séquence anti-sens capable de s'hybrider spécifiquement avec une séquence nucléotidiquement codant pour ladite protéine ou un anticorps dirigé contre ladite protéine pour la fabrication d'un médicament destiné à traiter des pathologies liées à un dysfonctionnement du système immunitaire.

La demande décrit également l'utilisation d'une séquence peptidique capable d'agir avec ladite protéine, ou des composés pharmaceutiques capables de contrôler l'expression de ladite protéine, et/ou de leurs partenaires et/ou de leurs interactions, pour la fabrication d'un médicament destiné à traiter des pathologies liées à un dysfonctionnement du système immunitaire.

La demande décrit par ailleurs une méthode de prévention et/ou traitement d'une pathologie liée à un dysfonctionnement du système immunitaire, cette méthode comprenant l'administration à un patient nécessitant un tel traitement d'une quantité thérapeutiquement efficace d'un agent modulateur de l'expression ou de l'activité d'une CRMP, en association avec un véhiculé pharmaceutiquement acceptable.

Ces modes de réalisation sont détaillés ci-après.

Plus précisément, la présente invention concerne un acide nucléique antisens capable d'hybrider avec un acide nucléique codant pour une protéine CRMP, ou anticorps anti-CRMP, pour son utilisation dans le traitement d'une pathologie impliquant un dysfonctionnement des lymphocytes T, ladite pathologie étant choisie parmi :
- les maladies à prions;
- les maladies neuroinflammatoires démyélinisantes; et
- la sclérose en plaques.

La présente invention a également pour objet une méthode de criblage *in vitro* de molécules utiles pour le traitement de maladies à prions, dans laquelle on met en contact une molécule à tester avec une protéine prion PrP et une protéine CRMP, et on évalue la capacité de la molécule à inhiber l'interaction de la CRMP, ou d'un dimère de CRMP, avec la protéine PrP, une action inhibitrice sur cette interaction étant indicative d'une molécule utile pour le traitement de maladies à prions.

La présente invention concerne également une méthode *in vitro* de pronostic et/ou de diagnostic d'une pathologie liée à un dysfonctionnement du système immunitaire, la méthode comprenant la mise en évidence dans des cellules du système immunitaire prélevées chez un patient, d'une expression ou localisation anormale d'une protéine CRMP par rapport à des cellules contrôles, dans laquelle lesdites cellules du système immunitaire sont des lymphocytes, ladite pathologie étant choisie parmi :
a. les maladies à prions;
b. la sclérose en plaques;
c. et les maladies neuroinflammatoires démyélinisantes.

### Pathologies visées :

Comme décrit dans la présente demande, les pathologies visées d'un point de vue thérapeutique ou diagnostique, sont liées à un dysfonctionnement du système immunitaire, et notamment à un dysfonctionnement de la prolifération des cellules du système immunitaire, en particulier des cellules T.

Plus précisément, ces pathologies comprennent :
- les leucémies T (en particulier celle de l'adulte) et les lymphomes (c'est-à-dire la prolifération maligne des cellules T) ;
- les infections virales, telles qu'une infection par le virus de l'herpès, de la rougeole, le virus d'Epstein-Barr, HTLV-1 (on parle alors d'atteintes neuro-inflammatoires associées à l'infection par le rétrovirus HTLV1, paraparésie spastique tropicale ou myélopathie associée à HTLV-1 - TSP/HAM), ou une infection par le virus VIH (virus du SIDA aussi appelé neurosida). Plus généralement, on vise toute infection virale qui peut résulter en une invasion du SNC par les lymphocytes T (encéphalite liée à la rougeole, etc).
- les maladies à prions.

Sont par ailleurs concernées les atteintes dysimmunes liées à la mutation de Fas et Fasl ; et les atteintes neuro-inflammatoires associées à une activation des lymphocytes.

Il est également envisageable de traiter des maladies auto-immunes (et notamment celles liées à Fas), en agissant via les protéines CRMPs et leurs différents partenaires biologiques, sur la maturation des lymphocytes T au niveau du thymus.

A titre représentatif de ces pathologies auto-immunes, on peut plus particulièrement citer l'arthrite rhumatoïde, la myasthénie, le lupus érythémateux, l'asthme, la sclérose en plaques, ou tout trouble immun impliquant une reconnaissance immune et la cible des cellules en soi ou des tissus et résultant dans une ou plusieurs réponses inflammatoires. La sclérose en plaques est la plus fréquente des maladies auto-immunes.

Plus généralement, l'invention concerne la thérapie ou le diagnostic des maladies neuroinflammatoires démyélinisantes.

Selon un mode préféré de l'invention, le patient est un humain, de préférence un adulte, mais la méthode de traitement de l'invention peut également être appliquée à des mammifères ou autres vertébrés.

### Modulation des CRMPs:

Conformément à la présente invention, on cherche à moduler l'activité ou l'expression des CRMPs. Cette modulation peut être directe ou indirecte.

Une modulation directe de l'activité protéique CRMP est une modulation qui est effectuée par action directe sur l'activité et/ou l'expression de la protéine elle-même. Des agents capables de moduler directement l'activité protéique CRMP sont soit des agonistes soit des antagonistes et peuvent aussi être désignés « activateurs directs » ou « inhibiteurs directs » respectivement.

Par « agonlste », on entend donc un agent qui augmente l'activité tandis qu'un antagoniste signifie un agent qui inhibe l'activité de la protéine.

Préférentiellement, de tels agonistes ou antagonistes sont capables de moduler l'interaction de la protéine CRMP avec des molécules endogènes qui agissent habituellement directement en amont ou en aval de la protéine CRMP dans une cascade de signalisation. Il peut s'agir par exemple d'une interaction CRMP-sémaphorine, CRMP-plexine, CRMP-kinase ou encore CRMP-protéine du cytosquelette.

De tels agents sont par exemple des anticorps dirigés contre la protéine CRMP ou des aptamères.

Une altération de l'interaction entre deux protéines CRMPs homologues ou hétérologues est un autre exemple de modulation de l'activité CRMP. Une interaction entre des protéines « homologues » signifie une interaction entre au moins deux mêmes types de protéines CRMP tels que par exemple des homodimères CRMP2-CRMP2.

L'interaction entre protéines CRMPs « hétérologues » signifie une interaction entre au moins deux protéines CRMPs différentes, par exemple des hétérodimères CRMP2-CRMP5.

Parmi les agents capables de moduler directement l'expression d'une CRMP, on peut citer des agents qui altèrent (c'est-à-dire augmentent ou diminuent) le niveau de production de la protéine CRMP. Ces agents peuvent être par exemple un polypeptide CRMP ou une séquence d'acide nucléique codant pour cette protéine, ou des agents capables de moduler la transfection et/ou la traduction des gènes CRMP telles que des séquences d'acide nucléique antisens ou ARN double brin inhibiteur.

Une modulation indirecte de l'activité protéique CRMP est une modulation qui est effectuée par action sur l'activité et l'expression d'agents endogènes extracellulaire ou intracellulaire qui agissent habituellement en amont (inducteur) ou en aval (effecteur) de la protéine CRMP comme cascade de signalisation. Un inducteur d'une protéine CRMP est par exemple une sémaphorine, en particulier la sémaphorine 3A (Sema 3A) ou la sémaphorine 4D (Sema 4D). Les exemples d'effecteurs incluent des tyrosine kinase, des GTPase de la famille Rho ou Rac, des transférases (transglutaminase).

Sont également intéresantes d'autres protéines capables d'interagir avec des protéines CRMPs, qui peuvent être identifiées dans des échantillons pathologiques, tels que du liquide céphalorachidien ou des biopsies de cerveau, chez un patient (humain ou animal) affecté par un désordre immunologique. Des agents permettant d'obtenir une modulation indirecte de activité ou de l'expression d'une CRMP peuvent être sélectionnés aisément par un homme du métier, par exemple à la lumière des types de modulateur direct décrit ci-dessus.

Comme divulgué dans la présente demande, et sauf autrement décrit, les termes « agent » ou « composé test » peuvent se référer à une ou plusieurs molécules structurellement définies telles que des polypeptides, oligonucléotides, molécule minérale ou organique, de nature endogène ou exogène. Ces agents peuvent également être des composés indéfinis tels que des extraits cellulaires, tissulaires, ou des liquides biologiques d'origine animale ou végétale.

L'utilisation de protéines CRMPs ou d'acide nucléique codant pour une CRMP est intéressante lorsque l'on cherche à accroître la quantité en au moins l'une des CRMPs. On peut également envisager d'utiliser un composé ou un mélange de composés d'origine synthétique ou naturelle qui active ou inhibe l'expression ou l'action de ces CRMPs.

A titre illustratif de ce type de composés, on peut plus particulièrement citer les molécules susceptibles de se comporter comme un partenaire biologique des CRMPs, en utilisant par exemple les CRMPs comme molécule de signalisation intracellulaire.

### Protéines CRMP :

Les protéines CRMPs plus particulièrement considérées selon l'invention sont les protéines CRMP1, CRMP2, CRMP3, CRMP4 et CRMP5.

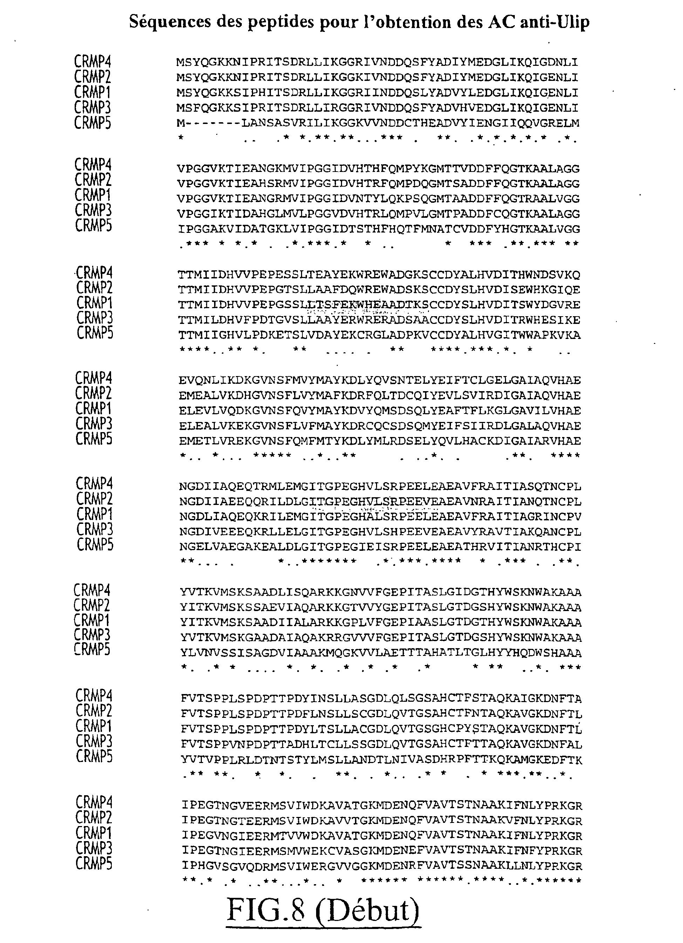

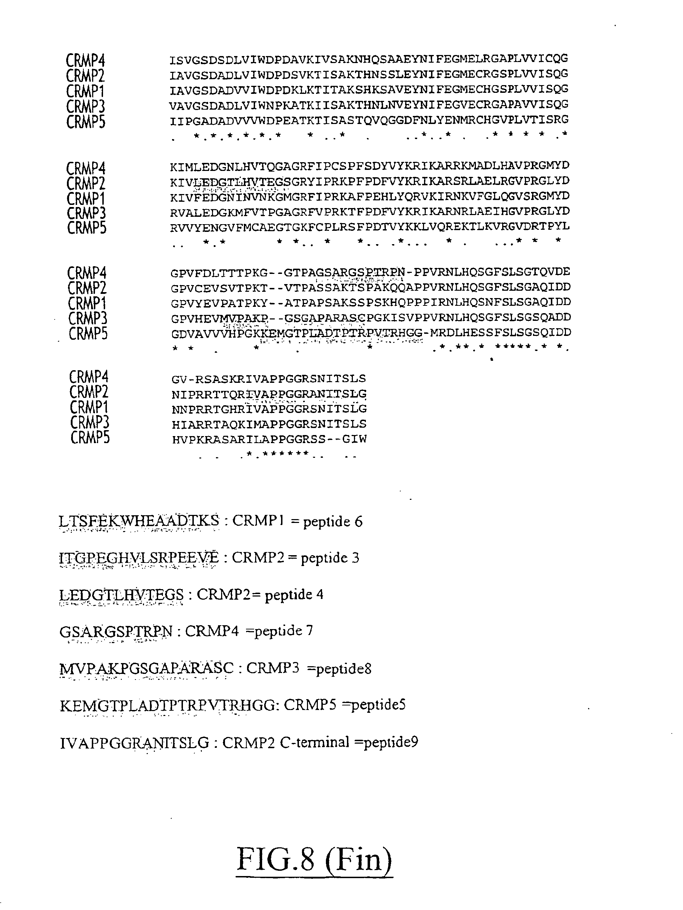

La présente invention concerne de manière préférentielle l'utilisation d'au moins une protéine CRMP choisie parmi les séquences d'acides aminés des protéines humaines CRMP1, CRMP2, CRMP3, CRMP4 et CRMP5 qui sont représentées en figure 8.

Selon la présente invention :
- La protéine CRMP1 fait notamment référence à une protéine comprenant la séquence d'acides aminés représentée en figure 8 ainsi que tous fragments polypeptidiques ou dérivés correspondants.
- La protéine CRMP2 fait notamment référence à une protéine comprenant la séquence d'acides aminés représentée en figure 8 ainsi que tous fragments polypeptides ou dérivés correspondants.
- La protéine CRMP3 fait notamment référence à une protéine comprenant la séquence d'acides aminés représentée en figure 8 ainsi que tous fragments polypeptides ou dérivés correspondants.
- La protéine CRMP4 fait notamment référence à une protéine comprenant la séquence d'acides aminés représentée en figure 8 ainsi que tous fragments polypeptides ou dérivés correspondants.
- La protéine CRMP5 fait notamment référence à une protéine comprenant la séquence d'acides aminés représentée en figure 8 ainsi que tous fragments polypeptides ou dérivés correspondants.

Les polypeptides dérivés font référence à toute variante polypeptidique des protéines ci-dessus ou toute autre molécule résultant d'une modification génétique et/ou de nature chimique de l'une des séquences précisées précédemment, c'est-à-dire obtenue par mutation, délétion, addition, substitution et/ou toute modification chimique d'un unique ou d'un nombre limité d'acides aminés, de même que toute séquence isoforme, ladite séquence dérivée, modifiée ou isomorphe ayant conservée au moins l'une des propriétés la rendant biologiquement active.

Sont ainsi comprises les séquences homologues, définies comme
i) les séquences similaires à au moins 70%, de préférence 80 %, de préférence encore 90 % des séquences humaines de CRMP (telles que représentées à la figure 8) ;
ii) les séquences codées par une séquence d'acide nucléique homologue c'est-à-dire une séquence d'acide nucléique hybridant avec une séquence codant pour les CRMPs humaines ou sa séquence complémentaire, dans des conditions stringentes d'hybridation.

Le terme "similaires" se réfère à la ressemblance parfaite ou identité entre les acides aminés comparés mais aussi à la ressemblance non parfaite que l'on qualifie de similitude. Cette recherche de similitudes dans une séquence polypeptidique prend en compte les substitutions conservatives qui sont des substitutions d'acides aminés de même classe, telles que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique) ; d'acides aminés aux chaînes latérales apolaires (tels que la glycine, l'alanine, la valine, la leucine, l'isoleucine, la proline, la phénylalanine, la méthionine, le tryptophane, et la cystéine).

Plus généralement, par « séquence d'acides aminés homologue », on entend donc toute séquence d'acides aminés qui diffère de la séquence humaine de la figure 8, par substitution, délétion et/ou insertion d'un acide aminé ou d'un nombre réduit d'acides aminés, notamment par substitution d'acides aminés naturels par des acides aminés non naturels ou pseudoacides aminés à des positions telles que ces modifications ne portent pas significativement atteinte à l'activité biologique de la CRMP.

L'homologie est généralement déterminée en utilisant un logiciel d'analyse de séquence (par exemple, Sequence Analysis Software Package of the Genetics Computer Group, University of Wisconsin Biotechnology Center, 1710 University Avenue, Madison, WI 53705). Des séquences d'acides aminés similaires sont alignées pour obtenir le maximum de degré d'homologie (i.e. identité ou similitude, comme défini plus haut). A cette fin, il peut être nécessaire d'introduire de manière artificielle des espaces (« gaps ») dans la séquence. Une fois l'alignement optimal réalisé, le degré d'homologie est établi par enregistrement de toutes les positions pour lesquelles les acides aminés des deux séquences comparées sont identiques, par rapport au nombre total de positions.

L' « activité biologique » des protéines CRMPs inclut toute propriété biologique de ces protéines. Par exemple, l'activité peut être déterminée en évaluant inhibition de croissance axonale en réponse aux sémaphorines, en particulier la Sema 3A ou la Sema4D et/ou en évaluant l'inhibition de la croissance des oligodendrocytes, et/ou encore la migration des cellules du système immunitaire en réponse aux sémaphorines. Sont aussi incluses les propriétés immunologiques des CRMPs, en particulier la capacité de provoquer la production d'anticorps, du type des anticorps anti-CV2 dans les SNP. Est également incluse une activité enzymatique CRMP directe ou indirecte, ou comme substrat d'une autre enzyme.

Sont également concernées les différentes formes de protéines considérées susceptibles d'être reconnues par leurs anticorps respectifs. Il peut ainsi s'agir de leurs formes dimériques, phosphorylées et/ou tronquées.

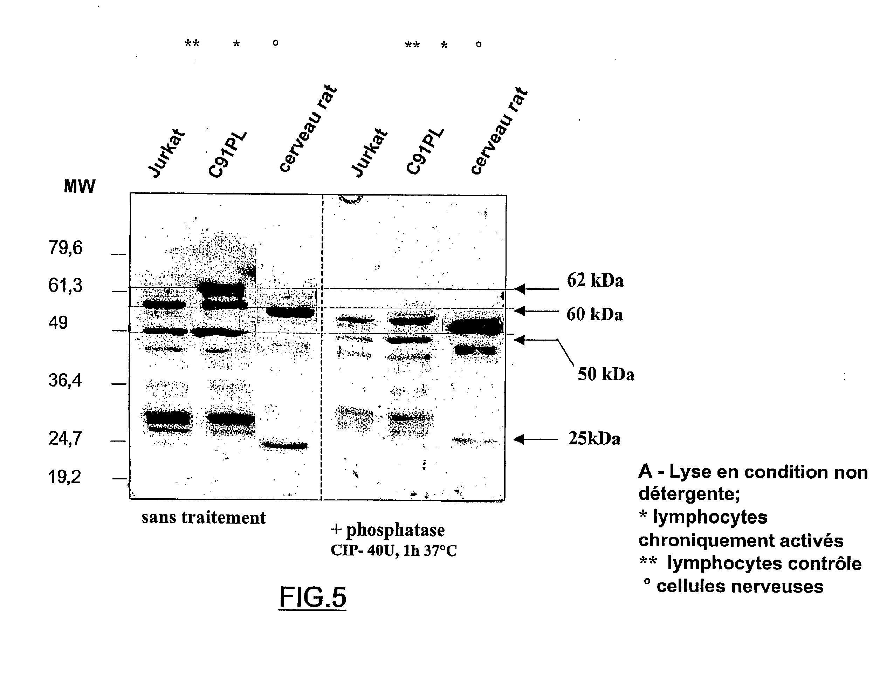

En figure 5, il est ainsi démontré par Western Blot à l'aide d'un anticorps spécifique la présence d'une forme particulière de CRMP2 phosphorylée dans les lymphocytes T hyperprolifératifs infectés par le rétrovirus HTLV1 (C91PL), comparés aux lymphocytes contrôles (Jurkat).

Sont plus particulièrement préférées dans le cadre de la présente invention à titre de cibles thérapeutiques, les protéines CRMP2 et CRMP5. La protéine CRMP5 semble notamment constituer une cible particulièrement intéressante pour le diagnostic et/ou le traitement des pathologies de type maladies à prions.

Les protéines CRMPs, seules ou en mélange, peuvent être associées à un véhicule pharmaceutiquement acceptable au sein d'une composition pharmaceutique, utile pour la prévention ou le traitement d'un dysfonctionnement du système immunitaire.

### Acides nucléiques :

La demande décrit également l'utilisation de toute séquence d'acides nucléiques isolés codant pour l'une des protéines CRMP1, CRMP2, CRMP3, CRMP4 et CRMP5, ou fragments nucléotidiques ou séquences dérivant de l'une des séquences, du fait de la dégénérescence du code génétique ou du fait de mutation, de délétion, ou d'insertion d'au moins un nucléotide, lesdites séquences dérivées ayant une activité biologique pratiquement identique à celle de la protéine considérée.

Les différentes séquences nucléotidiques ou peptidiques décrites dans la demande peuvent être d'origine artificielle ou non. Il peut s'agir de séquences d'ADN ou d'ARN, obtenues par criblage de banques de séquences au moyen de sondes élaborées sur la base des séquences d'origine. De telles banques peuvent être préparées par des techniques classiques de biologie moléculaire connues de l'homme de l'art.

Les séquences nucléotidiques décrites dans la demande peuvent également être préparées par synthèse chimique ou encore par des méthodes mixtes incluant la modification chimique ou enzymatique de séquences obtenues par criblage des bandes. Ces séquences nucléotidiques permettent la réalisation de sondes nucléotidiques capables de s'hybrider fortement et spécifiquement avec une séquence d'acides nucléiques, d'un ADN génomique ou d'un ARN messager codant pour un peptide selon l'invention ou un fragment biologiquement actif de celui-ci.

Sont comprises les séquences homologues, définies comme :
i) des séquences similaires à au moins 70 %, de préférence 80 %, de préférence encore 90 %, d'une séquence codant pour une CRMP humaine (telle que représentée à la figure 8) ; ou
ii) des séquences hybridant avec une séquence codant pour une CRMP humaine ou sa séquence complémentaire, dans des conditions stringentes d'hybridation.

De manière préférentielle, une telle séquence nucléotidique homologue hybride spécifiquement aux séquences complémentaires de la séquence codant pour une CRMP de la figure 8 dans des conditions stringentes. Les paramètres définissant les conditions de stringence dépendent de la température à laquelle 50% des brins appariés se séparent (Tm).

Pour les séquences comprenant plus de 30 bases, Tm est définie par la relation : Tm=81,5+0,41 (%G+C)+16,6Log(concentration en cations) - 0,63(%formamide) -(600/nombre de bases) (Sambrook *et al*., 1989). Pour les séquences de longueur inférieure à 30 bases, Tm est définie par la relation : Tm= 4(G+C) + 2 (A+T).

Dans des conditions de stringence appropriées, auxquelles les séquences aspécifiques n'hybrident pas, la température d'hybridation peut être de préférence de 5 à 10°C en dessous de Tm, et les tampons d'hybridation utilisés sont de préférence des solutions de force ionique élevée telle qu'une solution 6xSSC par exemple.

Le terme "séquences similaires" employé plus haut se réfère à la ressemblance parfaite ou identité entre les nucléotides comparés mais aussi à la ressemblance non parfaite que l'on qualifie de similitude. Cette recherche de similitudes dans les séquences nucléiques distingue par exemple les purines et les pyrimidines.

Parmi de telles séquences homologues, sont comprises les séquences des gènes de mammifères autres que l'homme, codant pour une CRMP, de préférence d'un primate, d'un bovin, ovin ou porc, ou encore d'un rongeur, ainsi que les variants alléliques.

Un acide nucléique codant pour une CRMP est notamment utile dans le cadre d'une thérapie génique, en association avec un véhicule pharmaceutiquement acceptable, au sein d'une composition pharmaceutique.

### Antisens :

L'utilisation de séquences anti-sens anti- CRMP1, CRMP2, CRMP3, CRMP4 et/ou CRMP5 est pour sa part privilégiée lorsque l'on cherche à bloquer l'expression de la protéine CRMP considérée.

Les séquences nucléotidiques codant pour les protéines CRMP1, CRMP2, CRMP3, CRMP4 ou CRMP5 sont utiles pour la production de séquences anti-sens capables de s'hybrider spécifiquement avec une séquence d'acides nucléiques incluant l'ARN messager qui peut être utilisé en thérapie génique.

La demande décrit donc également l'utilisation de séquences anti-sens capables d'inhiber au moins partiellement la production des protéines CRMPs identifiées ci-dessus.

Ces séquences peuvent être formulées dans des compositions pharmaceutiques, utiles en thérapie génique.

### Anticorps anti-CRMP :

La demande décrit également l'utilisation d'anticorps mono- ou polyclonaux ou leurs fragments, anticorps chimériques ou immunoconjugués obtenus à partir d'une CRMP choisies parmi les protéines CRMP1, CRMP2, CRMP3, CRMP4 ou CRMP5, dérivés ou fragments polypeptidiques biologiquement actifs de CRMPs.

Ces anticorps sont utiles pour bloquer l'expression de la protéine CRMP considérée.

Les anticorps polyclonaux peuvent être obtenus selon le protocole décrit dans les exemples. Ces anticorps peuvent toutefois être également obtenus à l'aide de méthodes conventionnelles.

Les anticorps polyclonaux peuvent être obtenus à partir du sérum d'un animal immunisé contre un polypeptide selon les modes opératoires usuels.

Plus particulièrement, on peut utiliser comme antigène un fragment peptidique approprié, tel que défini plus haut, pouvant être couplé par l'intermédiaire d'un résidu réactif à une protéine ou un autre peptide. Des lapins sont immunisés avec l'équivalent de 1 mg de l'antigène peptidique selon la procédure décrite par Benoit et al., PNAS USA, 79, 917-921 (1982). A des intervalles de quatre semaines, les animaux sont traités par des injections de 200 µg d'antigène et saignés 10 à 14 jours plus tard. Après la troisième injection, l'ant-sérum est examiné pour déterminer sa capacité à se lier au peptide antigène radiomarqué à l'iode, préparé par la méthode chloramine-T et est ensuite purifié par une chromatographie sur colonne échangeuse d'ion carboxyméthyl cellulose (CMC). Les molécules d'anticorps sont ensuite recueillies dans les mammifères et isolées jusqu'à la concentration souhaitée par les méthodes bien connues de l'homme de l'art, par exemple, en utilisant DEAE Sephadex pour obtenir la fraction IgG.

Afin d'augmenter la spécificité du sérum polyclonal, les anticorps peuvent être purifiés par une chromatographie d'immuno-affinité en utilisant des polypeptides immunisant en phase solide. L'anticorps est mis en contact avec le polypeptide immunisant en phase solide pendant une durée suffisante de façon à faire immuno-réagir le polypeptide avec la molécule d'anticorps afin de former un complexe immunologique en phase solide.

A titre représentatif des séquences utilisées pour produire ces anticorps, on peut particulièrement citer ceux représentés en figure 8.

Il s'agit de :
- peptide 6 (CMRP1) : SEQ ID n° 22 ;
- peptide 3 (CMRP2) : SEQ ID n° 23 ;
- peptide 4 (CMRP2) : SEQ ID n° 24 ;
- peptide 7 (CRMP4) : SEQ ID n° 25 ;
- peptide 8 (CRMP3) : SEQ ID n° 26 ;
- peptide 5 (CRMP5) : SEQ ID n° 27 ;
- peptide 9 (CRMP2 C-terminal) : SEQ ID n° 28.

L'anti-pep4 est plus spécifique de CRMP2, ce qui est particulièrement avantageux compte tenu de la forte homologie entre les protéines CRMPs qui rend difficile la production d'anticorps spécifiques. Le peptide comprend une sérine qui est un site de phosphorylation potentiel et qui, vraisemblablement, facilite la détection de la forme phosphorylée.

L'anti-pep 9 (CRMP2 C-terminal) est également spécifique d'une partie de la protéine CRMP2 qui est tronquée dans certaines circonstances pathologiques.

L'anti-pep5 est spécifique de CRMP5 et permet donc avantageusement de différencier CRMP5 des autres CRMPs.

On peut également utiliser des anticorps monoclonaux, qui peuvent par exemple être obtenus par la méthode classique de culture d'hybridomes de Köhler et Milstein, Nature, 256, 495-497, (1975).

Les anticorps peuvent être des anticorps chimériques, des anticorps humanisés, des fragments Fab et F(ab')2. Ils peuvent également se présenter sous forme d'immunoconjugués ou d'anticorps marqués.

Les anticorps anti-CRMP sont utiles en association avec un véhicule pharmaceutiquement acceptable dans des compositions pharmaceutiques utiles pour la prévention ou le traitement du dysfonctionnement du système immunitaire. Ils sont également utiles comme outils de diagnostic.

### Interaction avec d'autres partenaires :

On peut également envisager d'intervenir dans la cascade de signalisation en mettant en oeuvre un composé ou mélange de composés d'origine synthétique ou naturelle capable d'inhiber l'action desdites protéines CRMPs et plus particulièrement de bloquer l'interaction soit entre deux d'entre elles, soit entre l'une d'entre elles et ses partenaires biologiques naturels.

De manière plus générale, il est possible d'envisager également la mise en oeuvre des protéines CRMPs comme appâts pour identifier de nouvelles cibles thérapeutiques dans des extraits biologiques, de type lipidique ou biopsies, de patients atteints de maladies autoimmunes, neuroinflammatoires, lymphomes ou leucémies T.

La demande décrit également l'utilisation d'au moins une protéine CRMP à titre d'outil de diagnostic pour détecter, identifier et/ou doser au moins un partenaire biologique d'une protéine CRMP dans des extraits biologiques de patients atteints de maladies autoimmunes, neuroinflammatoires, lymphomes ou leucémies T.

Les inventeurs ont ainsi mis en évidence que les CRMPs, qui sont donc des molécules de signalisation intracellulaire, réagissaient avec la protéine prion PrP, impliquée dans la signalisation membranaire.

Vraisemblablement la modulation de l'expression des CRMPs dans les lymphocytes par l'intermédiaire de la protéine PrP interviendrait dans le processus de dégénérescence au niveau du transfert de l'information pathogène du système immunitaire vers le système nerveux central. Cet aspect de invention est illustré par l'exemple 4 ci-après.

En tant que partenaire biologique des CRMP, la protéine PrP peut également être une cible intéressante de modulation de l'activité des CRMP.

L'approche thérapeutique à considérer consisterait donc à bloquer l'interaction entre PrP et la protéine CRMP, et notamment CRMP5.

En l'occurrence, est décrite dans la demande une composition pharmaceutique comprenant précisément à titre de principe actif un composé ou mélange de composés, d'origine synthétique ou naturelle capable de cibler au moins une protéine CRMP et/ou d'inhiber l'interaction naturelle d'au moins une protéine CRMP et/ou un dimère de CRMP avec la protéine prion PrP.

De préference, la protéine CRMP considérée est la CRMP5.

Comme décrit ici, le composé ou mélange de composés agit plus particulièrement en bloquant l'interaction entre les deux protéines considérées. Conviennent tout particulièrement à ce titre les composés dits aptamères. Il s'agit de molécules qui possèdent la faculté de se lier à d'autres molécules avec une grande affinité et spécificité. A titre représentatif de ce type de composés, on peut plus particulièrement proposer les aptamères peptidiques.

Les pathologies susceptibles d'être traitées selon cette variante sont les maladies à prion, sporadique, acquise ou génétique ou, de manière plus générale, toute maladie impliquant la PrP dans la signalisation cellulaire.

En ce qui concerne les composés possédant une activité stimulatrice ou inhibitrice vis-à-vis des protéines CRMPs, ils peuvent être sélectionnés à l'aide d'une méthode de screening dans laquelle le composé à tester est mis en contact avec une protéine CRMP et l'interaction entre les deux protéines déterminées.

Un autre objet de l'invention est d'ailleurs une méthode de criblage *in vitro* de molécules utiles pour le traitement de maladies à prions, dans laquelle on met en contact une molécule à tester avec une protéine prion PrP et une protéine CRMP, et on évalue la capacité de la molécule à inhiber l'interaction de la CRMP, ou d'un dimère de CRMP, avec la protéine PrP, une action inhibitrice sur cette interaction étant indicative d'une molécule utile pour le traitement de maladies à prions.

### Administration :

Les modes d'administration, posologies et formes galéniques optimaux des compositions pharmaceutiques décrites dans la demande peuvent être déterminés selon les critères généralement pris en compte dans l'établissement d'un traitement thérapeutique adapté à un patient, comme par exemple l'âge ou le poids corporel du patient, la gravité de son état général, la tolérance au traitement, les effets secondaires constatés, etc.

Préférentiellement, les compositions pharmaceutiques décrites dans la demande peuvent être administrées par voie systémique, de préférence par voie intraveineuse, par voie intramusculaire, intradermique ou par voie orale.

De manière générale, une quantité thérapeutiquement ou prophylactiquement efficace variant d'environ 0,1 µg à environ 1 mg de protéine CMRP peut être administrée à des adultes humains.

La demande décrit également une composition pharmaceutique comprenant un acide nucléique tel que défini précédemment, codant pour une CMRP ou un acide nucléique antisens et un véhicule pharmaceutiquement acceptable, ladite composition étant destinée à être utilisée en thérapie génique. L'acide nucléique de préférence inséré dans un vecteur généralement viral (tels que les adénovirus et les rétrovirus) peut être administré sous forme nue, exempt de tout véhicule favorisant le transfert à la cellule cible, tels que des liposomes anioniques, des lipides cationiques, des microparticules, par exemple des microparticules d'or, des agents de précipitation, par exemple du phosphate de calcium, ou tout autre agent facilitant la transfection. Dans ce cas, le polynucléotide peut être simplement dilué dans une solution physiologiquement acceptable, telle qu'une solution stérile ou une solution stérile tampon, en présence ou en l'absence d'un véhicule.

De manière alternative, un acide nucléique tel que décrit dans la demande peut être associé à des agents qui facilitent la transfection. Il peut être, entre autres, (i) associé à un agent chimique qui modifie la perméabilité cellulaire tel que la bupivacaïne ; (ii) encapsulé dans des liposomes, éventuellement en présence de substances supplémentaires facilitant la transfection : ou (iii) associé à des lipides cationiques ou des microparticules de silice, d'or ou de tungsténe.

Lorsque les constructions d'acide nucléique décrites dans la demande recouvrent des microparticules, celles-ci peuvent être injectées par voie intradermique ou intraépidermique par la technique du canon à gènes, "gene gun" (WO 94/24263).

La quantité à utiliser comme médicament dépend notamment de la construction d'acide nucléique elle-même, de l'individu auquel cet acide nucléique est administré, du mode d'administration et du type de formulation, et de la pathologie. De manière générale, une quantité thérapeutiquement ou prophylactiquement efficace variant d'environ 0,1 µg à environ 1 mg, de préférence d'environ 1 µg à environ 800 µg et, de manière préférentielle d'environ 25 µg à environ 250 µg, peut être administrée à des adultes humains.

Les constructions d'acides nucléiques décrites dans la demande peuvent être administrées par toute voie d'administration conventionnelle telle que notamment par voie parentérale. Le choix de la voie d'administration dépend en particulier de la formulation choisie.

### Méthodes diagnostiques :

La demande décrit également une méthode *in vitro* de pronostic et/ou de diagnostic d'une pathologie liée à un dysfonctionnement du système immunitaire, la méthode comprenant la mise en évidence dans des cellules du système immunitaire, à savoir notamment des lymphocytes, cellules dendritiques et monocytes prélevées chez un patient, (par exemple dans le sang ou le cerveau) d'une expression ou localisation anormale d'une protéine CRMP par rapport à des lymphocytes contrôles.

La demande décrit plus précisément une méthode pour le pronostic et/ou diagnostic de maladies auto-immunes, de lymphomes, leucémie de l'adulte, maladies neuro-inflammatoires liées ou non à une infection virale et/ou de maladies à prions, caractérisée en ce que l'on met en évidence dans des lymphocytes prélevés chez un individu, la présence d'au moins une protéine CRMP dont l'expression, la séquence ou la localisation est modifiée par rapport à des lymphocytes contrôles obtenus chez des sujets sains.

Toute pathologie liée à un dysfonctionnement des lymphocytes T, telle que décrite plus haut, peut ainsi être diagnostiquée.

On peut en particulier déterminer si un individu est porteur ou est susceptible de développer une pathologie choisie parmi :
- les leucémies T et les lymphomes (T) ;
- les infections virales telle que virus de l'herpès, de la rougeole, le virus d'Epstein-Barr, HTLV-1 ou VIH ;
- les maladies à prions ;
- et les maladies neuroinflammatoires démyélinisantes. L'évaluation de l'expression des protéines CRMPs peut être effectuée par différentes techniques bien connues de l'homme de l'art. L'ARN codant pour les protéines peut être détecté et quantifié à l'aide de sondes nucléotidiques spécifiques.

La demande décrit ainsi une méthode de diagnostic *in vitro* d'une pathologie liée à un dysfonctionnement du système immunitaire ou d'une prédisposition à développer une pathologie liée à un dysfonctionnement du système immunitaire, comprenant les étapes consistant à :
- mettre en présence un échantillon biologique contenant de l'ARNm obtenu à partir de lymphocytes chez un patient, avec des oligonucléotides spécifiques permettant l'amplification de tout ou partie du transcrit du gène codant pour une CRMP ;
- amplifier ledit transcrit ;
- détecter et quantifier les produits d'amplification ;
   une modification du taux de transcrit de la CRMP par rapport au contrôle normal étant indicatrice d'une pathologie liée à un dysfonctionnement du système immunitaire ou d'une prédisposition à développer une telle pathologie.

Les produits des gènes codants pour les protéines CRMPs peuvent être également dosés à l'aide d'anticorps correspondants tels que définis ci-dessus et localisés dans la cellule.

La demande décrit également une méthode de diagnostic *in vitro* d'une pathologie liée à un dysfonctionnement du système immunitaire ou d'une prédisposition à développer une pathologie liée à un dysfonctionnement du système immunitaire, pour la détection ou la mesure du taux d'expression et/ou de l'activité de CRMP dans un échantillon de lymphocytes d'un patient, à l'aide d'anticorps anti-CRMP. Cette méthode comprend la mise en contact d'au moins un anticorps dirigé contre la CRMP avec ledit échantillon dans des conditions permettant la formation éventuelle de complexes immunologiques spécifiques entre la CRMP et le ou lesdits anticorps et la détection des complexes immunologiques spécifiques éventuellement formés et/ou l'inhibition de l'activité de la CRMP par l'anticorps.

Alternativement, la présence des anticorps anti-CRMP peut être déterminée à l'aide des protéines CRMPs ou de leurs fragments épitopiques qui peuvent être marqués de manière à ce que les complexes formés entre la protéines et lesdits anticorps soient ensuite facilement détectables dans des échantillons biologiques.

Les exemples et figures ci-après sont présentés à titre illustratif et non limitatif de la présente invention.

### FIGURES :

Figure 1 : Comparaison par analyse RT-PCR du niveau d'expression des ARNm codant CRMP -4, -2 et -1 dans des lymphocytes et cellules mononucléées, contrôles ou issues de patients atteints de pathologies dysimmunes. Le niveau d'expression des ARNm des CRMP est normalisé par rapport au niveau d'expression du gène-ubiquitaire GAPDH. Les résultats sont exprimés en valeur relative comme un rapport de pixels entre l'amplicon de chaque CRMP et l'amplicon de G3PDH.
Figure 2 : Profil électrophorétique des protéines CRMP1, 2 et 4 dans une lignée lymphocytaire Jurkat comparée à une lignée de cellule nerveuse Dev.
Figure 3 : Détection immunocytochimique de la location subcellulaire de la protéine CRMP2 dans des cellules mononucléées sanguines (PBL) de patients contrôle ou infectés par HTLV-1 ou présentant une déficience immune liée au système Fas/Fas ligand, ou infectés par le VIH.
Figures 4 : caractérisation de la présence nucléaire reconnue par l'anti CRMP-2 (peptide 4) dans les lymphocytes T hyperprolifératifs. Figures 4a : examen après immunodétection de CRMP-2 et contrecoloration par l'intercalant de l'ADN Dapi dans les cellules Jurkat T (contrôles) et les cellules C 8166 (T infectés par HTLV-1 non productrices de virus). Figures 4b : examen de l'immunodétection de CRMP-2 en microscopie confocale dans les cellules CEM (lignée T contrôle) et les cellules C91PL (lignée infectée par HTLV-1).
Figure 5 : Démonstration par Western Blot à l'aide de l'anticorps spécifique anti-CRMP2 (peptide 4) de la présence d'une forme particulière de CMRP2 phosphorylée dans les lymphocytes T hyperprolifératifs (C91 PL) comparé aux lymphocytes contrôles (Jurkat).
Figure 6 : Analyse immunohistochimique de l'expression des protéines CRMP5 et CRMP2 au niveau du thymus humain foetal (A, B, C) et dans un thymome (D, E, F).
Figure 7 : Caractérisation par essais double-hybride par conjugaison de l'interaction entre les protéines PrP bovine et CRMP5 humaine.
Figure 8 : Séquences en acides aminés des protéines humaines CRMP1, CRMP2, CRMP3, CRMP4 et CRMP5. Sont également présents sur cette figure les séquences des peptides choisis pour produire des anticorps spécifiquement dirigés contre les CRMPs.
La Figure 9 montre une cinétique de migration de lymphocytes T transfectés ou non avec un vecteur codant pour la CRMP-2.
La figure 10 rapporte le nombre de cellules T ayant migré dans trois expériences, après surexpression de CRMP-2 gfp.
La figure 11 rapporte le nombre de cellules T ayant migré dans deux expériences, après transfection de plasmides CRMP-2 associé à cmyc et CRMP-2 mutée (Delta 381CRMP2-cmyc).

### MATERIELS ET METHODES

### 1. Cellules et Culture cellulaire

● Cellules du SI : Les lymphocytes T sont soit des lymphocytes établis en lignées (IL-2 indépendante), soit des lymphocytes en culture primaire et dont la croissance nécessite la présence d'IL-2. Ces lymphocytes sont cultivés (37°C, 5% CO₂) en suspension dans un milieu salin supplémenté : soit en SVF (10%) dans le cas des lignées lymphocytaires, soit en sérum humain de type AB (SAB 10%) et en IL-2 dans le cas des cultures primaires de lymphocytes T. Des lymphocytes périphériques (PBL pour peripheral blood lymphocytes) ont également été fraîchement isolés à partir du sang de patients ou de sujets contrôle, séparés sur un gradient de Ficoll et récupérés après une phase d'adhésion des monocytes/macrophages.
● Traitements des lymphocytes T : Pour certaines expériences, les PBL fraîchement isolés ont été traités par des anticorps agonistes CD3 (10 µg/ml) qui miment l'activation des lymphocytes par un antigène via le récepteur T (TCR) ou par la Phytohémagglutinine (PHA - 10 µg/ml) (C. Malcus).

### 2. Examen des transcripts (ARNm) par la méthode de RT-PCR (reverse transcription et polymerase chain reaction).

● Isolement des ARN totaux : Cette expérience se fait systématiquement à 4°C pour éviter une dégradation des ARN par les Rnases. Les extractions d'ARN ont été effectuées à partir de cultures cellulaires ou de lymphocytes fraîchement isolés auxquels une solution de RNAzol est rajoutée (2 ml pour 10⁶ cellules). Le RNAzol contient le phénol et l'isothiocyanate de guanidium et lyse les cellules. La séparation de phase est effectuée à l'aide de chloroforme (400 µl pour 2 ml de RNAzol). Après homogénéisation (vortex) puis 5 min de repos, l'échantillon est centrifugé au repos (5 minutes) puis centrifugé à 12000 rpm pendant 30 minutes à 4°C. Les ARN présents dans la phase aqueuse (volume mesuré) sont précipités une première fois par addition d'un volume identique d'isopropanol (15 min à 4°C) puis centrifugation (12000 rpm pendant 15 minutes à 4°C). Une deuxième précipitation est réalisée en ajoutant au culot un volume d'acétate de sodium 0,3 M pH 5,2 ainsi que trois volumes d'éthanol absolu à -20°C. Le précipité est recueilli par centrifugation (12000 rpm, 15 minutes, 4°C). L'élimination des sels s'effectue par ajout au culot de 800 µl d'éthanol 80% à -20°C suivie d'une centrifugation. Après élimination de toute trace d'éthanol, le culot est dissout dans 100 µl d'eau distillée et stocké à -80°C. Le dosage des ARN se fait par mesure de la densité optique au spectrophotomètre à 260 et 280 nm (1 unité de DO à 260 nm = 40 µg d'ARN). Le rapport des DO obtenues à 260 nm et à 280 nm doit être proche de 2, indice d'une bonne extraction d'ARN sans trace de protéines.
● La transcription inverse : La transcription inverse (RT pour reverse transcription) est l'étape préliminaire à la polymérisation itérative qui utilise une ADN polymérase incapable de polymériser de l'ADN à partir d'ARN. La RT s'effectue grâce à une transcriptase inverse la Rtase du virus MuLV et est initiée par une amorce qui permet l'élongation et la synthèse d'un brin d'ADN complémentaire de l'ARN par l'enzyme. Dans une perspective d'étude comparative des produits d'amplification de différents ARNm, l'utilisation des amorces non-spécifiques (oligodT) s'est révélée plus adaptée, offrant la possibilité de mener les diverses PCR spécifiques à partir d'un même échantillon de RT.
500 ng d'ARN totaux dilués dans 7 µl d'eau (qsp) sont incubés 10 minutes à 70°C pour permettre la dénaturation des structures secondaires puis les tubes sont plongés immédiatement dans la glace pour éviter toute renaturation. A ces 70 µl d'ARN totaux sont alors ajoutés 1 µl d'aligodT (100 ng/µl) et 12 µl de milieu "d'incubation" contenant 0,5 nM de dNTP, 4 µl de tampon RT, 40 U de RNAsine (inhibiteur de Rnases), 10 mM de dithiotréitol ou DTT (dénaturant), 1 µl de MuLV-Rtase (200 U/µl). Le mélange réactionnel est incubé 90 minutes à 42°C. Les produits de RT sont ensuite dilués au 10/10^{ème} dans de l'eau distillée et stocké à -20°C.
● La réaction de polymérisation en chaîne (PCR) : La polymérisation itérative (PCR pour polymerase chain reaction) est une technique qui consiste à répéter des cycles de polymérisation d'un segment d'un ADN d'intérêt. La première étape de chaque cycle est une étape de dénaturation effectuée à 95°C. La deuxième étape est une étape d'hybridation ou accrochage avec des amorces spécifiques du segment qui encadrent la région à amplifier à une température à laquelle 50% des ADN sont sous forme double brin et les 50% restant sous la forme simple brin (Tm). La troisième étape est l'étape d'élongation à 72°C, température optimale pour l'activité de l'ADN polymérase ADN dépendante thermostable, la Taq polymerase. Les paramètres qui doivent être prioritairement pris en compte pour optimiser la PCR sont le choix des amorces (réalisés par le logiciel Primer3 et validés par comparaison avec les séquences humaines indexées, Blast), la température d'hybridation ou Tm, la concentration en MgCl₂ et le nombre de cycles. Toutes ces conditions ont été mises au point avant de réaliser les PCR et sont résumées dans le tableau suivant :

| | Amorces pour la RT-PCR Sonde pour le Southern-blot | | | Tm | [MgCl₂] | Nombre de cycles |
|---|---|---|---|---|---|---|
| CRMP-4 | **Sens** :gcaagtgtaggaaggcacgct | **Anti-sens :** ggcagctctggaacgtgaaga | **Sonde :** cctcagccttgttcttcacg | 62 °C | 2 mM | 28 |
| | **SEQ ID N°1** | **SEQ ID N°2** | **SEQ ID N°3** | | | |
| CRMP-2 | **Sens :** tcacatcagaactcctgtgg | **Anti-sens :** catgagtggaggaactttc | **Sonde :** ccatcaccacccttgtctct | 62 °C | 3 mM | 22 |
| | **SEQ ID N°4** | **SEQ ID N°5** | **SEQ ID N°6** | | | |
| CRMP-1 | **Sens :** tcatgctgaatccacctcgg | **Anti-sens :** cctctgaggcagttgacgg | **Sonde :** gacatcgccaaggactgact | 62 °C | 3 mM | 26 |
| | **SEQ ID N°7** | **SEQ ID N°8** | **SEQ ID N°9** | | | |
| CRMP-3 | **Sens :** gccgcccctaccagagacc | **Anti-sens :** gtgcagcgacagccagat | **Sonde :** cggagaaaacctcatcgt | 62 °C | 3 mM | 30 |
| | **SEQ ID N°10** | **SEQ ID N°11** | **SEQ ID N°12** | | | |
| CRMP-5 | **Sens :** ctgggagagaggagtggttg | **Anti-sens :** gaagtctcctccctggacct | **Sonde :** gttttgtggccgttaccagt | 62 °C | 3 mM | 28 |
| | **SEQ ID N°13** | **SEQ ID N°14** | **SEQ ID N°15** | | | |
| Actine | **Sens :** ggacttcgagcaagagatgg | **Anti-sens :** acatctgctggaaggtggac | **Sonde :** aagtactccgtgtgtggatcgg | 62 °C | 3 mM | 25 |
| | **SEQ ID N°16** | **SEQ ID N°17** | **SEQ ID N°18** | | | |
| G3PDH | **Sens :** ggctctccagaacatcatcc | **Anti-sens :** ggagattcagtgtggtgg | **Sonde :** gacatcaagaaggtggtgaagcag | 62 °C | 3 mM | 23 |
| | **SEQ ID N°19** | **SEQ ID N°20** | **SEQ ID N°21** | | | |

Chaque échantillon de PCR contient 10 µl de RT diluée au 1/10^{ème} et 40 µl de Mix (5 µl de tampon PCR 1X, 2 ou 3 µl de MgCl₂, 4x1 µl de dNTP, 1 µl de chaque amorce (sens et anti-sens), 0,4 µl de Taq polymérase, qsp 50 µl d'eau distillée. La PCR comprend une première étape de dénaturation de 5 min à 95°C puis un nombre de cycles déterminé pour chaque couple d'amorces (Cf. tableau) (1 min à 95°C, 1 min 15 à 62°C, 1 min 30 à 72°C). La PCR se termine par une étape d'élongation de 15 min à 72°C. les produits de PCR sont stockés à -20°C.
● Visualisation des RT-PCR : Southern-blot : Elle est réalisée après la séparation des produits d'amplification par électrophorèse puis transfert sur membrane et hybridation avec une sonde interne radiomarquée. La migration des différents produits de PCR se fait par dépôt de 10 µl de chaque échantillon sur un gel de 1,5% d'agarose dans un tampon TBE (Tris-Borate-EDTA) 0,5X contenant du Bromure d'éthidium (0,5 µg/ml au final) et application de 100 V. Une fois la migration effectuée, les ADNc sont transférés selon une méthode d'électrotransfert semi-sec sur membrane de nylon en tampon TBE 0,5X. Après transfert (15V-45 min), l'ADN est fixé sur la membrane par une solution de NaOH (0,4N-2 min) puis neutralisé (SSC 6X-10 min). La sonde interne, spécifique du fragment d'ADN amplifié par PCR (Cf. tableau) est radiomarquée en 5' par une terminale kinase (T4 Kinase) avec du γ32P-ATP. 2 µl sont incubés 10 min à 37°C avec 5 µl de tampon forward, 1 µl de T4 kinase, 2 µl de γ32P-ATP et 15 µl d'eau distillée. La kination est arrêtée à 4°C. la sonde radiomarquée est ensuite purifiée sur une colonne d'exclusion (Bio-Rad Laboratories) qui retient les nucléotides libres. Les membranes de transfert sont préhybridées 30 min à 42°C par incubation dans le milieu d'hybridation (SSC 6X, Denhardt 2X, tampon phosphate 25 mM, disodium éthylène diamine tétra-acétate ou EDTA 25 mM, SDS 0,1%, ADN de sperme de saumon 250 µg/ml) afin de saturer tous les sites non spécifiques. L'hybridation s'effectue pendant 30 min à 42°C avec la sonde interne. Les membranes sont ensuite lavées à 42°C dans des milieux de stringence croissante : dans du SSC6X/0,1% SDS 10 min, SSC 2X/SDS 0,1% 20 min et SSC 0,5X/0,1% SDS 10 min. Les membranes radiomarquées et scellées sous feuillet plastique sont ensuite placées dans une cassette d'autoradiographie munie d'un écran amplificateur. La visualisation des bandes d'ADNc radiomarquées se fait par lecture au Phosphorimager et la quantification par utilisation d'un logiciel informatique (Image Quant).

### 3. Immunodétection des protéines CRMP.

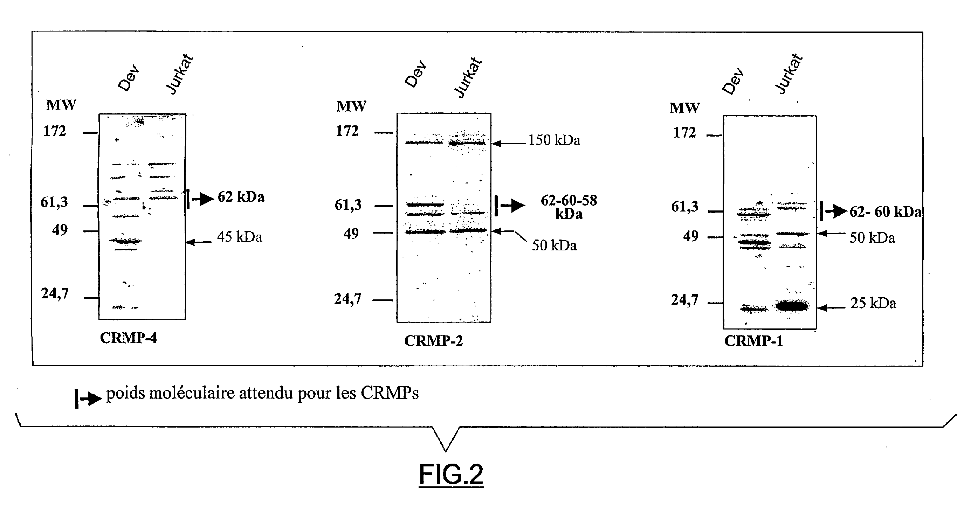

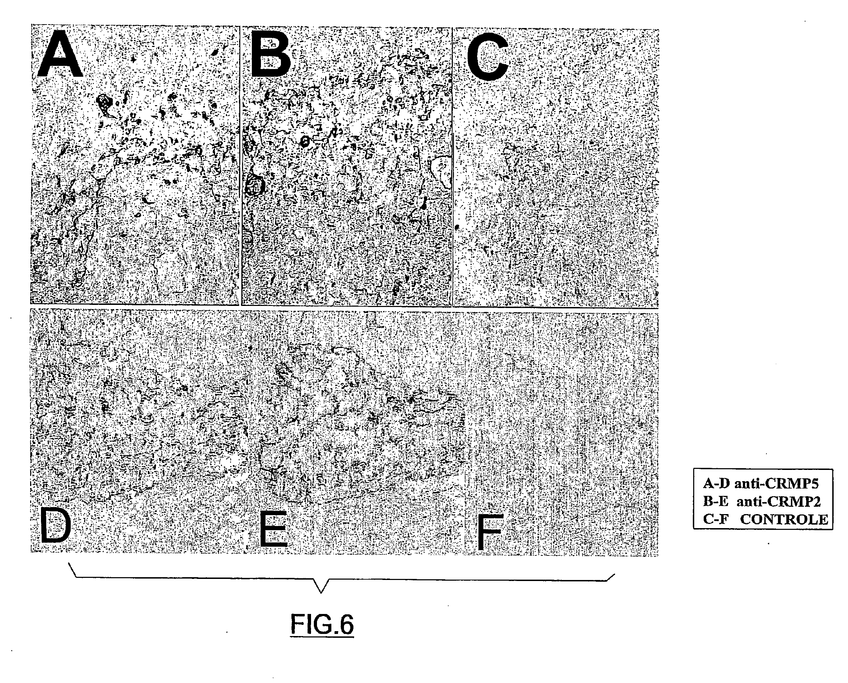

● Les anticorps anti-peptides : Des anticorps polyclonaux anti-peptides spécifiques des 5 CRMP ont été préparés après sélection d'un immunogène situé dans une zone non homologue pour les autres CRMP et injection à un lapin. La spécificité de chaque sérum pour une protéine CRMP donnée a été contrôlée par Western-blot sur les protéines CRMP recombinantes. Les sérums de chacun de ces lapins prélevés avant leur immunisation (sérums pré-immuns) sont négatifs en Western-blot sur les protéines recombinantes obtenues dans Ecolitranes formées ou cellules Hela transfectées et sont utilisés comme contrôle. On a au préalable déterminé les dilutions optimum pour leur utilisation en Western-blot ou en immunocytochimie.
● Western-blot : Cette technique consiste en la séparation électrophorétique des protéines puis transfert sur membrane qui permet la visualisation d'une protéine d'intérêt après la fixation d'un anticorps spécifique révélé par un anticorps secondaire couplé à la peroxydase. Les Western-blot sont réalisés sur extraits protéiques de lysats de cellules immunes ou nerveuses en culture ou fraîchement isolées. Les cellules sont en général lysées dans un tampon sans détergent (20 mM Tris-HCl, 10% sucrose, 1 mM d'EDTA, 5 mM d'EGTA et un cocktail d'anti-protéases (Complete^{™} 1X) et parfois en tampon RIPA (Tris-HCl 10 mM pH 7,2, 150 mM de NaCl, 1% de Triton 100X, 0,1% de SDS, 1 mM d'EDTA, 1% de sodium désoxycholate, Complete 1X). L'homogénéisation des lysats cellulaires est ensuite complétée par une sonication à 80Hz. Après un dosage protéique, une solution de 1 ou 2 µg/µl de protéines est conservée à -20°C. L'échantillon de dépôt (40 µg finaux de protéines) est dénaturé par l'addition d'un tampon réducteur (Tris-Hcl 0,0625 M pH 6,8, SDS 1 %, glycérol 10%, dithiothréitol DTT 0,1 M, bleu de bromophénol) et chauffage (5 min à 95°C). La séparation des protéines est réalisée sur gel d'acrylamide-SDS (10%-0,1%, respectivement). Après migration sous 100V, les protéines sont électrotransférées sur une membrane de nitrocellulose par transfert discontinu utilisant 3 tampons : Tampon 1 (0,3M Tris base, 20% Méthanol) ; Tampon 2 (25 mM Tris base, 20% Méthanol) ; Tampon 3 (25 mM Tris base, 40 mM EACA, 20% Méthanol) permettant un meilleur transfert de toutes les protéines, quel que soit leur poids moléculaire. Les membranes sont incubées 5 min dans une solution de Rouge Ponceau-acide trichloroacétique qui permet de fixer et visualiser les protéines. Les membranes sont ensuite saturées pendant 1 h à température ambiante dans une solution de Tampon phosphate (PBS)-Tween 20 0,1% - lait écrémé 5%. L'immunodétection des CRMP avec les sérums polyclonaux de lapin anti-CRMP dilués en PBS-Tween 20 0,1%-lait 1% (CRMP-4 : 1/100 ; CRMP-2 : 1/500 ; CRMP-1 : 1/500 ; CRMP-3 : 1/500 ; CRMP-5 : 1/200) ou pré-immun (1/200) est réalisée à 4°C- une nuit. Les blots sont alors rincés (3x5 min) en PBS-Tween 20 0,1 %-lait 1 % puis incubés 1 h avec un anticorps secondaire spécifique des IgG de lapin et couplé à la peroxydase (1/50000^{e}-1h-température ambiante). Après 3 rinçages (5 min) en PBS-Tween 20 0,1%-lait 1%, le blot est révélé en chambre noire grâce à un kit d'électrochimioluminescence (Covalab) et après impression sur film photographique.
   Lors d'une expérience, les échantillons ont été traités par une phosphatase pendant 1h à 37°C avant leur séparation sur un gel d'acrylamide-SDS : 2 µl de phosphatase alcaline CIP (Calf intestine phosphatase à 20 unités/µl) sont ajoutés à 20 µl d'échantillon protéique et à 2 µl de tampon permettant l'activité de l'enzyme.
● Immunocytochimie (ICC) : La détection des CRMP a été réalisée à l'échelle cellulaire par immunocytochimie. Les lymphocytes, cellules non adhérentes, sont étalés par centrifugation (cytospin- 600 rpm-5 min) alors que les cellules nerveuses Dev sont cultivées sur des lames de Permanox (Labteck). Une fixation des cellules par l'acétone (-20°C, 5 min) a pour but de fixer les protéines et de perméabiliser les cellules à l'entrée des anticorps réactionnels. Ces conditions ont permis la détection des CRMP 1, 2, 3, 4 et 5. Le blocage des sites non spécifiques se fait avec une solution de BSA 0,1% (30 minutes à température ambiante). Un premier contact des cellules fixées sur lames est effectué avec l'anticorps primaire (anti-CRMP produits chez le lapin dilués en PBS pH 7,4) (1h-37°C en chambre humide). Après 3 lavages en tampon phosphate (PBS pH 7,4, 5 min), l'anticorps secondaire spécifique des IgG de lapin est mis en contact (1h - température ambiante) et rincé 3 fois en PBS 5 min. La contrecoloration des noyaux se fait ensuite par une incubation en solution de Dapi (intercalant nucléique-0,025 µg/ml-1min). Les lames sont montées en glycérol tamponné pH 7,4. Les résultats de ces analyses sont représentés en sur les figures 2-8.

### EXEMPLE 1 :

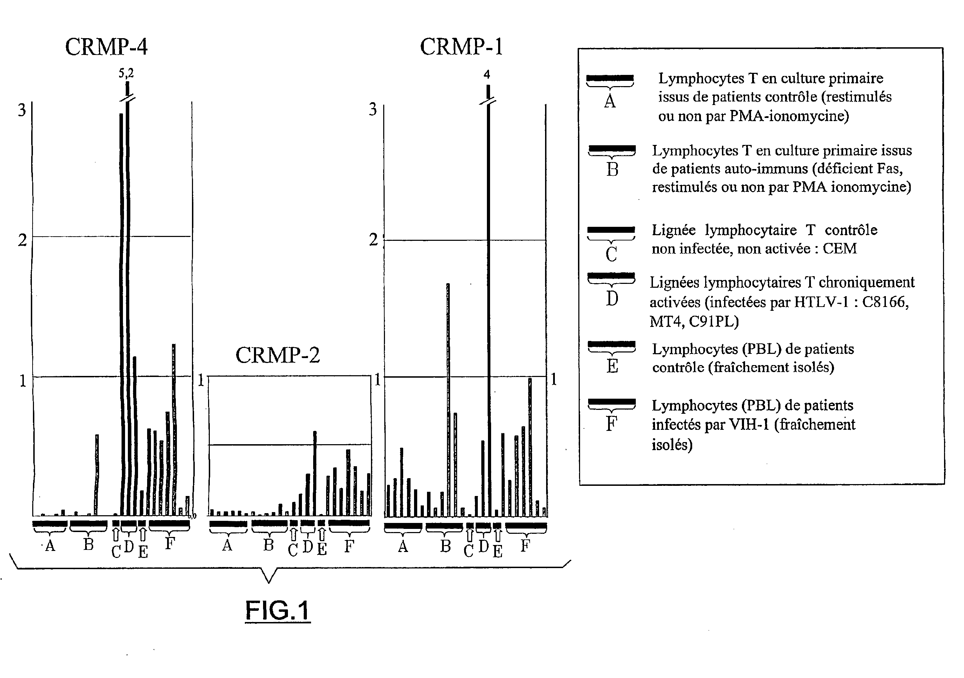

Evaluation de l'expression des CRMP 1, 2 et 4 dans les lymphocytes et cellules mononucléées, contrôles ou issues de patients atteints de pathologies dysimmunes.
● ARNm (par rapport à GAPDH)
Une première évaluation est réalisée en titrant les ARNm correspondants. Les résultats sont présentés de manière détaillée en figure 1 et résumés dans le tableau I ci-après.
Profil comparatif, en considérant tous les échantillons pour une même CRMP :

**TABLEAU I**

| | CRMP2 | CRMP4 | CRMP1 |
|---|---|---|---|
| Lignées T control | + | 0 | 0 |
| Lymphocytes T/HTLV-1 | ++ | +++ | +++ |
| Lympho. Patients "control" | ± | 0 | ± |
| Lympho. Patients défic. Apopt. | ± | 0 | +/++ |
| Patients VIH | ++ | ++ | ++ |

L'analyse précédente est complétée par détection des protéines CRMP1, 2 et 4 dans des lymphocytes T et des cellules nerveuses par Western Blot sur lysat cellulaire (Dev : cellules nerveuses ; Jurkat : lignée lymphocytaire T) en tampon non détergent et en utilisant les anticorps polyclonaux antipeptide spécifiques de chaque CRMP.

Les résultats sont présentés en figure 2.

On note que les anti CRMP-1, -2 et -4 détectent plusieurs isoformes partagées par les lymphocytes T et les cellules nerveuses.

### EXEMPLE 2 :

Détection immunocytochimique de la localisation subcellulaire de la protéine CRMP2 dans des cellules mononucléées sanguines (PBL) de patients contrôles ou infectés par le HTLV-1 ou présentant une déficience immune liée au système Fas/Fas ligand ou infectés par le VIH.

L'anticorps anti CRMP-2 a été au préalable préparé et isolé selon le protocole décrit au point 3 du préambule « matériels et méthode ».

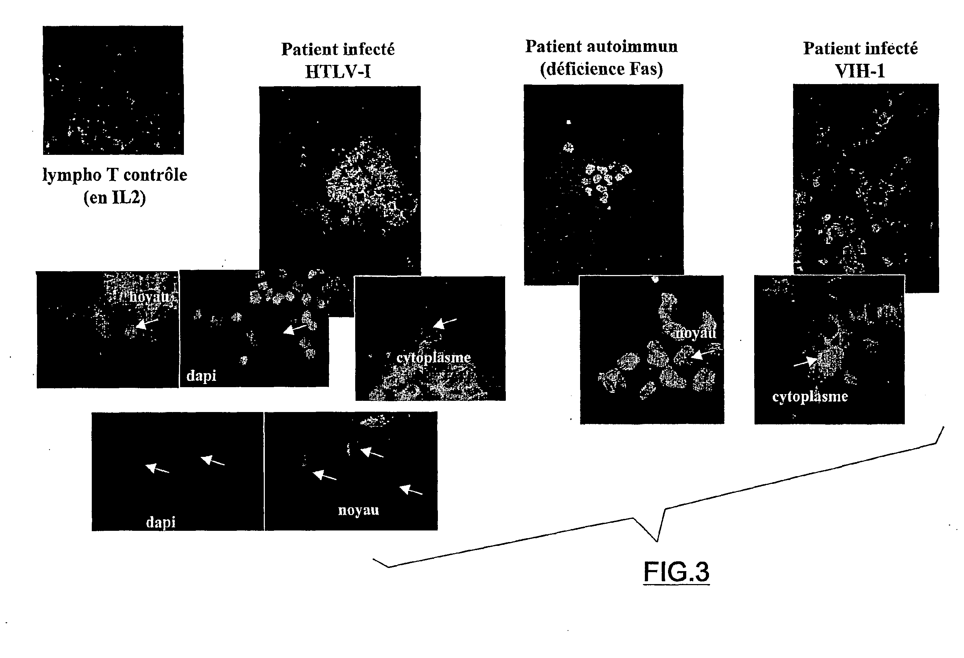

Les résultats sont illustrés en figure 3.

On note que :
- la localisation de CRMP-2 est essentiellement cytoplasmique dans des lymphocytes (PBL) isolés d'un sujet contrôle ou d'un sujet infecté par VIH-1 ; elle peut aussi être nucléaire dans les lymphocytes isolés de patients infectés par HTLV-1 ou dans les PBL d'un patient dysimmun (déficience Fas) ;
- CRMP-2 est plus exprimée dans les lymphocytes des patients comparés à ceux du sujet contrôle cultivés en IL2, donc préactivés.

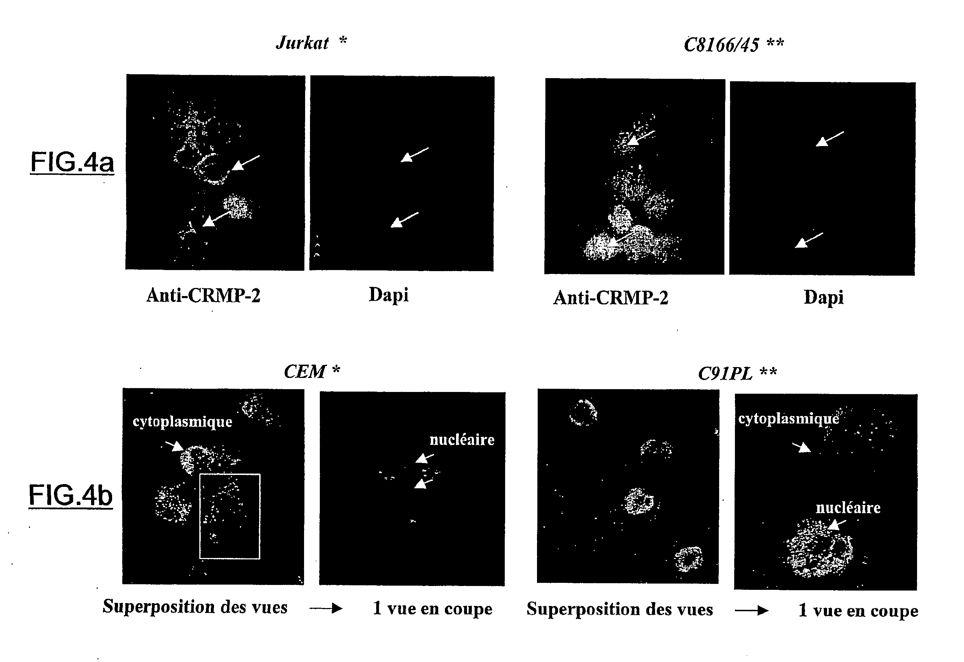

Par examen après immunodétection de CRMP-2 et contrecoloration par l'intercalant de l'ADN Dapi, on note que l'expression est majoritairement nucléaire dans les lymphocytes T chroniquement infectés par HTLV-1 et hyperprolifératifs. Quant à l'examen de l'immunodétection de CRMP-2 en microscopie confocale, il révèle une présence nucléaire très faible dans les lymphocytes T contrôle et majoritaire dans les lymphocytes T chroniquement infectés par HTLV-1 (figure 4).

En conclusion, L'expression de CRMP2 est augmentée dans les noyaux des lymphocytes prolifératifs. En revanche chez les patients infectés par le VIH, CRMP2 reste cytoplasmique. Chez les patients dysimmuns (déficients en Fas), la localisation est nucléaire dans quelques cellules. Les figures 3 et 4 rendent compte des résultats obtenus.

De même, la présence nucléaire d'une protéine reconnue par ce même anticorps a été vérifiée dans les lymphocytes T hyperprolifératifs par Western Blot sur des fractionnements cellulaires enrichis en noyaux.

### EXEMPLE 3 :

Caractérisation par immunohistochimie de l'expression des protéines CRMP5 et CRMP2 au niveau du thymus.

Les protéines ont été caractérisées à l'aide de leurs anticorps respectifs.

Les cellules analysées sont des cellules de thymus normal de foetus humain et dans un thymome chez un patient adulte.

A titre témoin, la même immunohistochimie a été réalisée sur du thymus humain d'un embryon de 6 semaines. Le thymus est surgelé et coupé au cryostat (15 µm). Les coupes sont fixées pendant 15 minutes dans l'acétone. L'immunohistochimie est réalisée dans les mêmes conditions que sur du cerveau.

Cette analyse révèle une expression de CRMP2 et CRMP5 dans les cellules épithéliales thymiques normale au stade embryonnaire. L'expression disparaît chez l'adulte normal et peut être induite dans le cas de pathologie tumorale (thymome).

### EXEMPLE 4 :

Caractérisation de l'interaction entre les protéines PrP bovine et CRMP5 humaine.

L'interaction entre les protéines PrP bovine et CRMP5 humaine a été caractérisée par essais double-hybride par conjugaisons.

Les ADNc codant pour ces deux protéines (complètes ou différentes formes dérivées) ont été clonés dans un vecteur "d'appât"et un vecteur "de proie". Le vecteur d'appât pEG202 (Gyuris et al, 1993, Cell 75, 791-803), comporte le gène codant pour le marqueur HIS3, une origine de réplication 2 µ et dirige l'expression de protéines fusionnées au domaine de liaison à l'ADN de la protéine LexA (1-202), sous le contrôle du promoteur constitutif ADHp. En ce qui concerne le vecteur de proie, pJG 4-5 (US 5,580,736), il comporte le gène codant pour le marqueur TRP1, une origine de réplication 2 microns et dirige l'expression de protéines fusionnées à une séquence de localisation nucléaire (NLS), au domaine activateur de transcription B42 et à l'épitope HA, sous le contrôle du promoteur inductible GALp. Par commodité, la fusion NLS-B42-HA est ici désignée "Act".

La souche EGY42 (*MATα*) (Golemis et al, 1992, Mol. Cell. Biol. 12, 3006-3014) a été co-transformée avec le vecteur plasmidique pSH18-34 (US 5,695,941) comportant le gène marqueur URA3 et huit opérateurs LexA placés en amont du gène rapporteur *lacZ*γ et différents vecteurs d'appâts dirigeant l'expression de LexA, LexA-PrP, LexA-PrPm (mutations sur PrP restant à déterminer), LexA-PrPtr (troncation de PrP restant à déterminer), LexA-MAX (contrôle négatif) et LexA-CRMP5.

La souche EGY48 (MAT α) a été transformée avec différents vecteurs de proie dirigeant l'expression de Act, Act-PrP, Act-BaxΔTM (contrôle négatif) et Act-CRMP5.

Ces différentes souches ont été conjuguées de façon à créer une matrice d'interaction.

Cette matrice a été répliquée sur milieu indicateur (Ura⁻, His⁻, Trp⁻, Galactose/X-gal) permettant d'exprimer les appâts et les proies, de sélectionner les exconjuguants diploïdes et de révéler les phénotypes d'interaction (couleur bleue, révélant une activité β-galactosidase observée lorsque le gène rapporteur *lacZ* est transcrit).

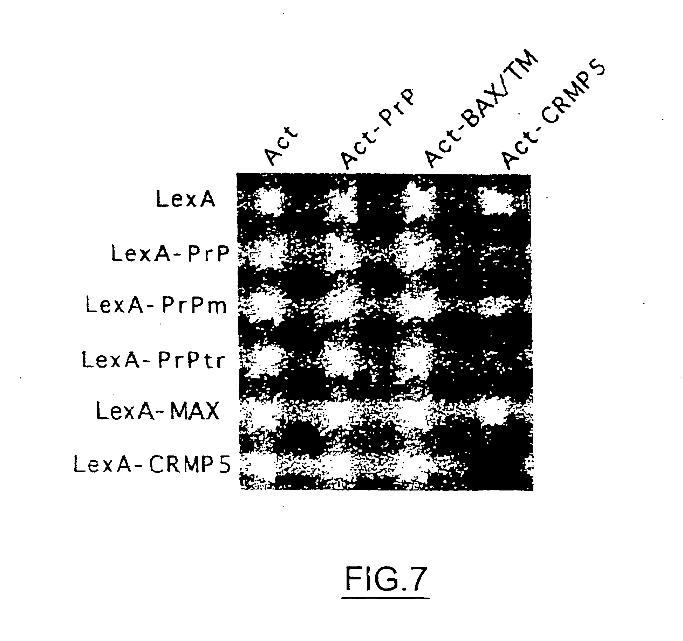

La figure 7 rend compte des résultats obtenus.

Les constructions testées sont les suivantes :
PrP : protéine PrP bovine (AA 22-237),
PrPm : protéine PrP bovine (AA 22-237) contenant diverses mutations restant à déterminer,
PrPtr : protéine PrP bovine (AA-22-?), tronquée vers le milieu de sa séquence (la position du codon stop reste à déterminer),
MAX : contrôle négatif d'interaction
CRMP 5 : protéine CRMP 5 humaine complète.
BAXΔTM : contrôle négatif d'interaction.

L'interaction entre les protéines PrP (différentes constructions détaillées ci-dessous) et la protéine CRMP5 est détectée lorsque les protéines PrP sont exprimées en tant qu'appâts et la protéine CRMP5 est exprimée en tant que proie. LexA-CRMP5 et Act-Prp ne donnent pas de phénotype d'interaction. Les contrôles négatifs (LexA-MAX et Act-BaxΔTM ainsi que LexA et Act) ne donnent aucun phénotype d'interaction avec les protéines PrP et CRMP. L'homodimérisation de CRMP5 fournit le contrôle positif d'interaction de cette matrice.

### EXEMPLE 5 :

Effet des CRMP sur la migration des lymphocytes T.

### Méthode

La migration des lymphocytes T (Jurkat) est évaluée après transfection ou non de plasmide codant différentes formes de CRMP-2 : CRMP2 associée gfp ; CRMP-2 associée cmyc et CRMP-2 mutée, Delta381 CRMP-2 (délétion des acides aminés 381 à 572 contenant sur un site TyrKin, un SH3 binding site et le site d'adhésion aux hétérodimères de tubuline (Fukata et al. Nat Cell Biol, 2002, Aug. 4(8):583-91). La transfection est vérifiée par la détection de GFP et de cmyc dans les cellules avant et après migration par Western Blot.

La migration s'effectue dans une chambre de Boyden (pores de 3 microns) pendant 18 heures dans le milieu RPMI standard + sérum de veau foetal. 400 000 cellules sont déposées dans la cupule du haut et le nombre de cellules ayant migré dans la cupule du bas est compté (cupules en triplicate, 3 expériences avec CRMP-2gfp et deux avec CRMP2 cmyc). Le niveau basal de migration est celui obtenu avec les cellules transfectées par un plasmide ne contenant pas l'insert CRMP-2.

L'association CRMP/vimentine est visualisée par immunoprécipitation et Western Blot (IP vimentine Wester CRMP) et co-immunodétection sur cellules sur lymphocytes T migrant ou non (microscopie confocale).

### Résultats

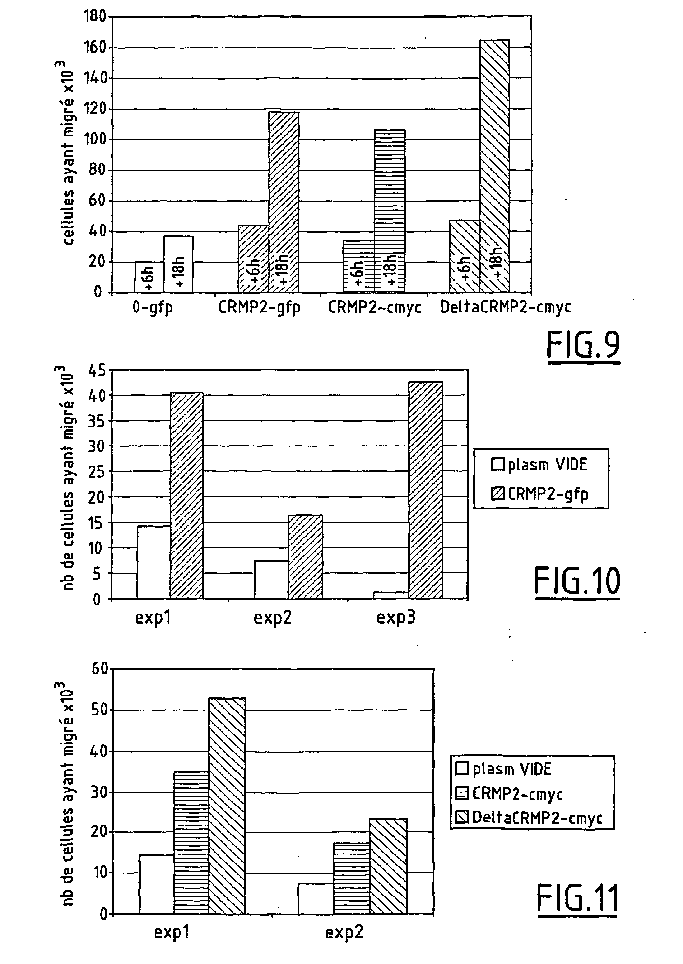

La surexpression de CRMP-2 gfp ou CRMP-2 cmyc dans les Jurkat augmente le nombre de cellules ayant migré en 6 heures et 18 heures (Figures 9 et 10).

La mutation de CRMP-2 Delta381 (site contenant TyrKin et le tubulin binding site) amplifie la migration suggérant un rôle de ce domaine dans la migration. (Figure 11).

La CRMP-2 est partenaire de la vimentine (protéine du cytosquelette) suggérant la participation de CRMP-2 dans le remaniement du cytosquelette.

## Revendications

1. Acide nucléique antisens capable d'hybrider avec un acide nucléique codant pour une protéine CRMP, ou anticorps anti-CRMP, pour son utilisation dans le traitement d'une pathologie impliquant un dysfonctionnement des lymphocytes T, ladite pathologie étant choisie parmi :
- les maladies à prions;
- les maladies neuroinflammatoires démyélinisantes; et
- la sclérose en plaques.

2. Acide nucléique antisens ou anticorps anti-CRMP pour son utilisation selon la revendication 1, dans laquelle la protéine CRMP est CRMP2 ou CRMP5.

3. Méthode de criblage *in vitro* de molécules utiles pour le traitement de maladies à prions, dans laquelle on met en contact une molécule à tester avec une protéine prion PrP et une protéine CRMP, et on évalue la capacité de la molécule à inhiber l'interaction de la CRMP, ou d'un dimère de CRMP, avec la protéine PrP, une action inhibitrice sur cette interaction étant indicative d'une molécule utile pour le traitement de maladies à prions.

4. Méthode *in vitro* de pronostic et /ou de diagnostic d'une pathologie liée à un dysfonctionnement du système immunitaire, la méthode comprenant la mise en évidence dans des cellules du système immunitaire prélevées chez un patient, d'une expression ou localisation anormale d'une protéine CRMP par rapport à des cellules contrôles, dans laquelle lesdites cellules du système immunitaire sont des lymphocytes, ladite pathologie étant choisie parmi :
a. les maladies à prions;
b. la sclérose en plaques;
c. et les maladies neuroinflammatoires démyélinisantes.

5. Méthode selon la revendication 4, ladite pathologie étant la sclérose en plaques.

6. Méthode selon la revendication 4, ladite pathologie étant une maladie neuroinflammatoire démyélinisante.

7. Méthode selon l'une quelconque des revendications 4 à 6, dans laquelle la protéine CRMP est CRMP2 ou CRMP5.

8. Méthode selon l'une quelconque des revendications 4 à 6, dans laquelle l'expression ou la localisation anormale de la protéine CRMP est associée à une phosphorylation modifiée de la protéine CRMP par rapport à des cellules contrôle, et dans laquelle la protéine CRMP est CRMP2.

9. Méthode selon l'une quelconque des revendications 4 à 8, dans laquelle la mise en évidence d'une expression ou localisation anormale de la protéine CRMP est réalisée à l'aide d'anticorps anti-CRMP.

10. Méthode selon la revendication 9, dans laquelle ledit anticorps anti-CRMP est un anticorps dirigé contre un peptide de séquence SEQ ID No 24, SEQ ID No 28 ou SEQ ID No 27.

## Claims

1. Antisense nucleic acid capable of hybridising with a nucleic acid encoding a CRMP protein, or anti-CRMP antibody, for use for the treatment of a pathology involving a dysfunction of the T lymphocytes, said pathology being selected from:
- prion diseases;
- demyelinating neuroinflammatory diseases; and
- multiple sclerosis.

2. Antisense nucleic acid or anti-CRMP antibody for its use according to claim 1, wherein the CRMP protein is CRMP 2 or CRMP5.

3. Method for the *in vitro* screening of molecules which can be used for the treatment of prion diseases, wherein a molecule to be tested is contacted with a PrP prion protein and a CRMP protein, and the capacity of the molecule to inhibit the interaction of the CRMP or of a CRMP dimer with the PrP protein is evaluated, and inhibitory action on this interaction being indicative of a molecule which may be used for the treatment of prion diseases.

4. Method for *in vitro* prognosis and/or diagnosis of a pathology associated with a dysfunction of the immune system, the method involving exhibiting, in cells of the immune system taken from a patient, an abnormal expression or location of a CRMP protein relative to control cells, wherein said cells of the immune system are lymphocytes, said pathology being selected from:
a. prion diseases;
b. multiple sclerosis, and
c. demyelinating neuroinflammatory diseases.

5. Method according to claim 4, said pathology being multiple sclerosis.

6. Method according to claim 4, said pathology being a demyelinating neuroinflammatory disease.

7. Method according to any one claims 4 to 6, wherein the CRMP protein is CRMP2 or CRMP5.

8. Method according to any one of claims 4 to 6, wherein the abnormal expression or location of the CRMP protein is associated with a modified phosphorylation of the CRMP protein relative to control cells, and wherein the CRMP protein is CRMP2.

9. Method according to any one of claims 4 to 8, wherein exhibiting an abnormal expression or location is carried out with anti-CRMP antibodies.

10. Method according to claim 9, wherein said anti-CRMP antibody is an antibody directed against a peptide of sequence SEQ ID NO: 24, SEQ ID NO: 28 or SEQ ID NO: 27.

## Patentansprüche

1. Antisense-Nukleinsäure, die in der Lage ist, mit einer für ein CRMP-Protein codierenden Nukleinsäure zu hybridisieren, oder Anti-CRMP-Antikörper, zur Verwendung bei der Behandlung einer Pathologie, die eine Funktionsstörung der T-Lymphozyten impliziert, wobei die Pathologie ausgewählt ist aus:
- Prionenerkrankungen,
- demyelinisierenden neuroinflammatorischen Erkrankungen und
- multipler Sklerose.

2. Antisense-Nukleinsäure oder Anti-CRMP-Antikörper zur Verwendung gemäß Anspruch 1, wobei das CRMP-Protein CRMP2 oder CRMP5 ist.

3. Verfahren zum In-Vitro-Selektieren von Molekülen, die für die Behandlung von Prionenerkrankungen von Nutzen sind, wobei ein zu testendes Molekül mit einem PrP-Prionenprotein und einem CRMP-Protein in Kontakt gebracht wird und die Fähigkeit des Moleküls bewertet wird, die Interaktion des CRMP oder eines CRMP-Dimers mit dem PrP-Protein zu hemmen, wobei eine Hemmwirkung auf die Interaktion auf ein Molekül hindeutet, das für die Behandlung von Prionenerkrankungen von Nutzen ist.

4. Verfahren für die In-Vitro-Prognose und/oder
- Diagnose einer Pathologie in Verbindung mit einer Funktionsstörung des Immunsystems, wobei das Verfahren das Nachweisen einer abnormalen Expression oder Lokalisation eines CRMP-Proteins in von einem Patienten entnommenen Zellen des Immunsystems im Verhältnis zu Kontrollzellen aufweist, wobei die Zellen des Immunsystems Lymphozyten sind, wobei die Pathologie ausgewählt ist aus:
a. Prionenerkrankungen
b. multipler Sklerose,
c. und demyelinisierenden neuroinflammatorischen Erkrankungen.

5. Verfahren gemäß Anspruch 4, wobei die Pathologie multiple Sklerose ist.

6. Verfahren gemäß Anspruch 4, wobei die Pathologie eine demyelinisierende neuroinflammatorische Erkrankung ist.

7. Verfahren gemäß einem der Ansprüche 4 bis 6, wobei das CRMP-Protein CRMP2 oder CRMP5 ist.

8. Verfahren gemäß einem der Ansprüche 4 bis 6, wobei die abnormale Expression oder Lokalisation des CRMP-Proteins mit einer modifizierten Phosphorylierung des CRMP-Proteins im Verhältnis zu Kontrollzellen verbunden ist, und wobei das CRMP-Protein CRMP2 ist.

9. Verfahren gemäß einem der Ansprüche 4 bis 8, wobei das Nachweisen einer abnormalen Expression oder Lokalisation des CRMP-Proteins mit Hilfe von Anti-CRMP-Antikörpern durchgeführt wird.

10. Verfahren gemäß Anspruch 9, wobei der Anti-CRMP-Antikörper ein Antikörper ist, der gegen ein Peptid der Sequenz SEQ ID Nr. 24, SEQ ID Nr. 28 oder SEQ ID Nr. 27 gerichtet ist.


### LISTE DE SEQUENCES

<110> INSERM
<120> Utilisation d'une protéine de la famille des CRMPs pour le traitement des maladies liées au système immunitaire
<130> BET 02/0808
<140> FR 0113342 <141> 2001-10-16
<160> 28
<170> PatentIn Ver. 2.1
<210> 1
   <211> 21
   <212> ADN
   <213> Séquence artificielle
<220>
   <223> Description de la séquence artificielle: oligonucléotide
<400> 1
   gcaagtgtag gaaggcacgc t 21
<210> 2
   <211> 21
   <212> ADN
   <213> Séquence artificielle
<220>
   <223> Description de la séquence artificielle: oligonucléotide
<400> 2
   ggcagctctg gaacgtgaag a 21
<210> 3
   <211> 20
   <212> ADN
   <213> Séquence artificielle
<220>
   <223> Description de la séquence artificielle: oligonucléotide
<400> 3
   cctcagcctt gttcttcacg 20
<210> 4
   <211> 20
   <212> ADN
   <213> Séquence artificielle
<220>
   <223> Description de la séquence artificielle: oligonucléotide
<400> 4
   tcacatcaga actcctgtgg 20
<210> 5
   <211> 19
   <212> ADN
   <213> Séquence artificielle
<220>
   <223> Description de la séquence artificielle: oligonucléotide
<400> 5
   catgagtgga ggaactttc 19
<210> 6
   <211> 20
   <212> ADN
   <213> Séquence artificielle
<220>
<223> Description de la séquence artificielle: oligonucléotide
<400> 6
   ccatcaccac ccttgtctct 20
<210> 7
   <211> 20
   <212> ADN
   <213> Séquence artificielle
<220>
   <223> Description de la séquence artificielle: oligonucléotide
<400> 7
   tcatgctgaa tccacctcgg 20
<210> 8
   <211> 19
   <212> ADN
   <213> Séquence artificielle
<220>
   <223> Description de la séquence artificielle: oligonucléotide
<400> 8
   cctctgaggc agttgacgg 19
<210> 9
   <211> 20
   <212> ADN
   <213> Séquence artificielle
<220>
   <223> Description de la séquence artificielle: oligonucléotide
<400> 9
   gacatcgcca aggactgact 20
<210> 10
   <211> 19
   <212> ADN
   <213> Séquence artificielle
<220>
   <223> Description de la séquence artificielle: oligonucléotide
<400> 10
   gccgccccta ccagagacc 19
<210> 11
   <211> 18
   <212> ADN
   <213> Séquence artificielle
<220>
   <223> Description de la séquence artificielle: oligonucléotide
<400> 11
   gtgcagcgac agccagat 18
<210> 12
   <211> 18
   <212> ADN
   <213> Séquence artificielle
<220>
   <223> Description de la séquence artificielle: oligonucléotide
<400> 12
   cggagaaaac ctcatcgt 18
<210> 13
   <211> 20
   <212> ADN
   <213> Séquence artificielle
<220>
   <223> Description de la séquence artificielle: oligonucléotide
<400> 13
   ctgggagaga ggagtggttg 20
<210> 14
   <211> 20
   <212> ADN
   <213> Séquence artificielle
<220> <223> Description de la séquence artificielle: oligonucléotide
<400> 14
   gaagtctcct ccctggacct 20
<210> 15
   <211> 20
   <212> ADN
   <213> Séquence artificielle
<220>
   <223> Description de la séquence artificielle: oligonucléotide
<400> 15
   gttttgtggc cgttaccagt 20
<210> 16
   <211> 20
   <212> ADN
   <213> Séquence artificielle
<220>
   <223> Description de la séquence artificielle: oligonucléotide
<400> 16
   ggacttcgag caagagatgg 20
<210> 17
   <211> 20
   <212> ADN
   <213> Séquence artificielle
<220>
   <223> Description de la séquence artificielle: oligonucléotide
<400> 17
   acatctgctg gaaggtggac 20
<210> 18
   <211> 22
   <212> ADN
   <213> Séquence artificielle
<220>
   <223> Description de la séquence artificielle: oligonucléotide
<400> 18
   aagtactccg tgtgtggatc gg 22
<210> 19
   <211> 20
   <212> ADN
   <213> Séquence artificielle
<220>
   <223> Description de la séquence artificielle: oligonucléotide
<400> 19
   ggctctccag aacatcatcc 20
<210> 20
   <211> 18
   <212> ADN
   <213> Séquence artificielle
<220>
   <223> Description de la séquence artificielle: oligonucléotide
<400> 20
   ggagattcag tgtggtgg 18
<210> 21
   <211> 24
   <212> ADN
   <213> Séquence artificielle
<220>
   <223> Description de la séquence artificielle: oligonucléotide
<400> 21
   gacatcaaga aggtggtgaa gcag 24
<210> 22
   <211> 15
   <212> PRT
   <213> Séquence artificielle
<220>
   <223> Description de la séquence artificielle: peptide
<400> 22
   Leu Thr Ser Phe Glu Lys Trp His Glu Ala Ala Asp Thr Lys Ser
   1 5 10 15
<210> 23
   <211> 16
   <212> PRT
   <213> Séquence artificielle
<220>
   <223> Description de la séquence artificielle: peptide
<400> 23
   Ile Thr Gly Pro Glu Gly His Val Leu Ser Arg Pro Glu Glu Val Glu
   1 5 10 15
<210> 24
   <211> 12
   <212> PRT
   <213> Séquence artificielle
<220>
   <223> Description de la séquence artificielle: peptide
<400> 24
   Leu Glu Asp Gly Thr Leu His Val Thr Glu Gly Ser
   1 5 10
<210> 25
   <211> 11
   <212> PRT
   <213> Séquence artificielle
<220>
   <223> Description de la séquence artificielle: peptide
<400> 25
   Gly Ser Ala Arg Gly Ser Pro Thr Arg Pro Asn
   1 5 10
<210> 26
   <211> 16
   <212> PRT
   <213> Séquence artificielle
<220>
   <223> Description de la séquence artificielle: peptide
<400> 26
   Met Val Pro Ala Lys Pro Gly Ser Gly Ala Pro Ala Arg Ala Ser Cys
   1 5 10 15
<210> 27
   <211> 20
   <212> PRT
   <213> Séquence artificielle
<220>
   <223> Description de la séquence artificielle: peptide
<400> 27
   Lys Glu Met Gly Thr Pro Leu Ala Asp Thr Pro Thr Arg Pro Val Thr
   1 5 10 15
   Arg His Gly Gly
   20
<210> 28
   <211> 15
   <212> PRT
   <213> Séquence artificielle
<220>
   <223> Description de la séquence artificielle: peptide
<400> 28
   Ile Val Ala Pro Pro Gly Gly Arg Ala Asn Ile Thr Ser Leu Gly
   1 5 10 15